# NEUE EUROPÄISCHE PATENTSCHRIFT

(11) **EP 3 695 733 B2**
(45) Veröffentlichungstag und Bekanntmachung des Hinweises auf die Entscheidung über den Einspruch: **29.07.2026**
(45) Hinweis auf die Patenterteilung: 10.08.2022
(21) Anmeldenummer: 20157632.9
(22) Anmeldetag: 17.02.2020
(51) Int. Cl.: A24F 40/46, A24F 40/485, A61M 11/04, A61M 15/06, A61M 16/00, H05B 3/20, H05B 3/04, H05B 3/44, A24F 40/10, A61M 15/00

(54) **VERDAMPFEREINSATZ FÜR EINEN INHALATOR, INSBESONDERE EIN ELEKTRONISCHES ZIGARETTENPRODUKT, VERDAMPFER-TANK-EINHEIT UND EIN ELEKTRONISCHES ZIGARETTENPRODUKT**
EVAPORATOR INSERT FOR AN INHALER, IN PARTICULAR AN ELECTRONIC CIGARETTE PRODUCT, EVAPORATOR TANK UNIT AND AN ELECTRONIC CIGARETTE PRODUCT
INSERT D'ÉVAPORATEUR POUR UN INHALATEUR, EN PARTICULIER UN PRODUIT DE CIGARETTE ÉLECTRONIQUE, UNITÉ DE RÉSERVOIR D'ÉVAPORATEUR ET PRODUIT DE CIGARETTE ÉLECTRONIQUE

(30) Priorität: 18.02.2019 DE 102019103990
(43) Veröffentlichungstag der Anmeldung: 19.08.2020
(60) Teilanmeldung: 21156170.9
(73) Patentinhaber: Körber Technologies GmbH, 21033 Hamburg (DE)
(72) Erfinder: CORNILS, Lasse, 22605 Hamburg (DE); ROMMING, Niklas, 22303 Hamburg (DE); JAKLIN, Jan, 79098 Freiburg (DE); NIEBUHR, Gunnar, 22605 Hamburg (DE); ULLNER, Tim, 21039 Hamburg (DE); MÜLLER, Thomas, 20259 Hamburg (DE)
(74) Vertreter: Müller Verweyen

(56) Entgegenhaltungen:
- EP-A1- 2 925 395
- EP-A1- 3 104 724
- EP-A1- 3 434 118
- WO-A1-2013/116572
- WO-A1-2016/138665
- WO-A1-2016/145611
- WO-A1-2017/163052
- WO-A1-2018/001102
- WO-A1-2020/169467
- WO-A1-2020/254388
- WO-A1-2020/254390
- WO-A1-2021/048039
- CN-U- 204 104 843
- CN-U- 205 671 482
- DE-A1- 102016 120 803
- GB-A- 2 515 562
- US-A1- 2016 007 653
- US-A1- 2016 106 153
- US-A1- 2016 331 026
- US-A1- 2017 367 402
- US-A1- 2018 343 923
- US-B1- 8 528 569
- US-B1- 9 795 169

## Beschreibung

Die vorliegende Erfindung betrifft einen Verdampfereinsatz für ein elektronisches Zigarettenprodukt, umfassend mindestens einen elektrischen Verdampfer zum Verdampfen von dem Verdampfer zugeführter Flüssigkeit und Zuführen der verdampften Flüssigkeit zu einem Luftstrom, ein Grundteil mit einer Mantelseite, die einen von dem Luftstrom durchströmbaren Luftstromkanal umschließt, wobei an der Mantelseite des Grundteils mindestens eine Flüssigkeitsöffnung zum Zuführen von verdampfbarer Flüssigkeit von außen in den Verdampfereinsatz zu dem Verdampfer angeordnet ist. Die vorliegenden abhängigen Ansprüche betreffen auch eine Verdampfer-Tank-Einheit und ein elektronisches Zigarettenprodukt, jeweils mit einem erfindungsgemäßen Verdampfereinsatz.

Herkömmliche elektronische Zigarettenprodukte bzw. Inhalatoren basieren auf der Docht-Wendel-Technologie. Durch Kapillarkräfte wird die Flüssigkeit aus einem Flüssigkeitsspeicher entlang eines Dochts so weit transportiert, bis die Flüssigkeit durch einen elektrisch beheizbaren Wendel erhitzt und somit verdampft wird. Der Docht dient als flüssigkeitsleitende Verbindung zwischen dem Flüssigkeitsspeicher und dem als Verdampfer dienenden Heizwendel. Ein Nachteil der Docht-Wendel-Technologie ist, dass eine mangelnde Versorgung von Flüssigkeit zu einer lokalen Überhitzung führen kann, wodurch Schadstoffe freigegeben werden können. Diesen sogenannten "Dry Puff" gilt es zu vermeiden.

US 9,795,169 B1 offenbart einen Verdampfereinsatz nach dem Oberbegriff von Anspruch 1.

Es ist die Aufgabe der Erfindung einen verbesserten Verdampfereinsatz bereitzustellen, der insbesondere eine einfache und zuverlässige Montage erlaubt.

Die Aufgabe wird gelöst durch die Merkmale im kennzeichnenden Teil des unabhängigen Anspruchs 1.

Erfindungsgemäß wird vorgeschlagen, dass der Verdampfereinsatz einen Träger zur Halterung des Verdampfers umfasst, um den Verdampfer mechanisch stabil halten zu können. Durch die Halterung des Verdampfers auf dem vom Grundteil verschiedenen beziehungsweise separaten Träger kann der Träger mit dem Verdampfer unabhängig vom Grundteil bereitgestellt werden, um zu dem erfindungsgemäßen Verdampfereinsatz zusammengeführt zu werden. Dies vereinfacht die Montage des Verdampfereinsatzes und die Bereitstellung des Trägers und des Grundteils als separate Komponenten. Erfindungsgemäß weist der Träger eine Durchgangsöffnung auf, die zur Flüssigkeitsversorgung des Verdampfers mit der Flüssigkeitsöffnung korrespondiert. Die Durchgangsöffnung verbindet die Flüssigkeitsöffnung mit dem Verdampfer flüssigkeitsleitend, um eine flüssigkeitsleitende Anbindung des Verdampfers bzw. des Verdampfereinsatzes an einen externen Flüssigkeitsspeicher zu begünstigen. Die Durchgangsöffnung weist vorteilhaft etwa die Größe einer Grundfläche des Verdampfers, der Fläche einer Einlassseite des Verdampfers, auf der Flüssigkeit in den Verdampfer eintreten kann, und/oder der Flüssigkeitsöffnung auf. Die Durchgangsöffnung ist vorzugsweise an der Einlassseite des Verdampfers vorgesehen. Dies erlaubt eine möglichst einfache Anordnung der Bauteile des Verdampfereinsatzes, insbesondere ist damit eine mantelseitige Anordnung des Verdampfers möglich.

Vorteilhaft bildet der Träger einen Luftkanal zum Führen des Luftstroms aus, um eine zuverlässige Leitung und Produktion von Aerosol bzw. Dampf zu begünstigen und einen integrierten Träger bereitzustellen, mit dem für die Verdampfung wichtige Faktoren vorteilhaft in dem Verdampfereinsatz integriert bestimmt werden können. Der Träger bildet insbesondere an der Auslassseite des Verdampfers den Luftkanal aus, der von dem Luftstrom, dem die verdampfte Flüssigkeit zugegeben wird, durchströmt oder durchströmbar ist. Dies führt zu einer zuverlässigen Produktion von Aerosol bzw. Dampf. Durch den Luftkanal strömt somit die mit verdampfter Flüssigkeit angereicherte Luft in Form eines Aerosols und/oder Dampfes.

Der Luftkanal kann als trägerbezogener Kanalabschnitt des vom Grundteil ausgebildeten Luftstromkanals aufgefasst werden. Der Luftstromkanal kann wenigstens teilweise stromaufwärts des Verdampfers in den Luftkanal münden beziehungsweise übergehen und/oder der Luftkanal kann wenigstens teilweise stromabwärts des Verdampfers in den Luftstromkanal münden beziehungsweise übergehen.

Vorzugsweise ist der Luftkanal zwischen dem Träger und der Wandung des Verdampfereinsatzes und/oder innerhalb des Trägers ausgebildet. Die Kontur beziehungsweise Form, insbesondere der Querschnitt des Trägers kann teilweise so ausgebildet sein, dass sich zwischen der Wandung des Verdampfereinsatzes und dem Träger der Luftkanal bildet, um einen besonders einfachen Aufbau des Trägers zu begünstigen. Der Luftkanal kann beispielsweise innerhalb des Trägers ausgebildet sein, um eine besonders zuverlässige und für die Aerosolqualität und -menge günstige Formgebung des Luftkanals zu begünstigen, die unabhängig von der Form der Wandung des Grundteils ist.

Der erfindungsgemäße Verdampfereinsatz ermöglicht besonders vorteilhaft die Bereitstellung eines Komplettverdampfers, der eine verlässliche und reproduzierbare Verdampfung gewährleistet und gleichzeitig einfach in oder an einen Inhalator beliebiger Bauform adaptierbar ist.

In einer bevorzugten Ausführungsform umfasst oder trägt der Träger eine elektrische Kontaktierung zum elektrischen Kontaktieren des Verdampfereinsatzes und/oder eine elektrische Leitung, um einen multifunktionalen Träger bereitstellen zu können. Die von dem Träger vorteilhaft umfassten elektrischen Kontakte dienen einer reproduzierbaren elektrischen Kontaktierung eines externen Teils, beispielsweise eines Basisteils, insbesondere zur Stromversorgung, des Inhalators. Damit kann der Verdampfer von dem externen Teil mit elektrischer Energie versorgt und/oder mittels Steuersignalen gesteuert werden. Der Verdampfer beziehungsweise der Verdampfereinsatz kann Signale an das externe Teil übermitteln. Die elektrische Kontaktierung kann beispielsweise metallische Elemente, Kontaktfedern und/oder Metallstifte umfassen, die beispielsweise von dem Träger getragen sind.

Die elektrische Leitung oder die elektrischen Leitungen können der Versorgung von elektrischen Komponenten in und/oder auf dem Träger, wie beispielsweise dem Verdampfer und/oder einer elektronischen Steuereinrichtung, dienen. Die elektrische Leitung weist vorteilhaft einen möglichst geringen elektrischen Widerstand auf, um elektrische Verlustleistung zu vermindern. Auf diese Weise ist es möglich, die Verlustleistung im System auf beispielsweise unter 500 mW zu reduzieren. Die Ausformung des Trägers kann so angepasst werden, dass die Leitungen beispielsweise in dem Träger verlaufen und beispielsweise eingegossen beziehungsweise vom Träger umfasst sind. Die elektrische Leitung oder die elektrischen Leitungen können von einem den Träger bildenden Kunststoff umspritzt sein, und/oder auf einer Oberfläche des Trägers verlaufen und somit vom Träger getragen sein.

In einer bevorzugten Ausführungsform sind die elektrischen Kontakte an einer Stirnseite des Verdampfereinsatzes angeordnet, um eine einfache elektrische Verbindung mit einem externen Bauteil, beispielsweise einem Komponententeil mit einem Energiespeicher herstellen zu können. Die Stirnseiten bezeichnen die Seiten, zwischen denen sich die Mantelseite des Grundteils erstreckt.

Vorteilhaft ist der Träger an einer Wandung des Grundteils abgestützt, um den Träger besonders einfach und mechanisch stabil halten zu können. Dafür weist der Träger vorteilhaft wenigstens teilweise eine Form, insbesondere einen Querschnitt auf, die dem Innenquerschnitt der Wandung des Grundteils entspricht. Beispielsweise weist das Grundteil einen Montageschacht auf, der durch die Wandung begrenzt ist, und in den der Träger eingesetzt werden kann, um sich an der Wandung abzustützen. Der Träger kann zur Halterung in dem Grundteil Rasten, Ausnehmungen und/oder Klemmungen aufweisen und/oder in das Grundteil geklebt werden.

In einer bevorzugten Ausführungsform bildet die Mantelseite wenigstens teilweise die Wandung, um einen einfachen Aufbau zu ermöglichen. Somit ist der Träger in Inneren des Grundteils angeordnet. Die in und/oder auf dem Träger angeordneten Komponenten sind somit gekapselt und insbesondere elektrische Komponenten sind vor Flüssigkeit geschützt.

In einer vorteilhaften Ausführungsform bildet der Träger einen vom Luftkanal verschiedenen Nebenluftkanal zum Führen eines Nebenluftstroms aus, um die Qualität des erzeugten Dampfes bzw. Aerosols zu verbessern. Der Nebenluftkanal beginnt vorzugsweise stromaufwärts des Verdampfers. Vorteilhaft wird der Nebenluftstrom dem Hauptluftstrom stromabwärts des Verdampfers zugeführt. Dazu können der Nebenluftkanal und der Luftkanal stromabwärts des Verdampfers zusammengeführt werden. Der Nebenluftkanal kann vorteilhaft im Träger beginnen und/oder vorteilhaft im Träger mit dem Luftkanal zusammengeführt werden. Es sind jedoch auch Ausführungsformen denkbar, in dem der Nebenluftkanal bereits stromaufwärts des Trägers beginnt und/oder stromabwärts des Trägers in den Luftkanal mündet.

Vorteilhaft umfasst der Träger zur Halterung des Verdampfers ein keramisches Substrat, um den Träger zur Halterung des Verdampfers thermisch stabil auszubilden und/oder gegebenenfalls den Verdampfer thermisch von dem Träger zu entkoppeln. Das keramische Substrat ist chemisch und mechanisch stabil gegenüber der im Betrieb des Verdampfers auftretenden Temperaturen von beispielsweise bis zu 300 °C und thermischen Lastwechseln, die beispielsweise ca. 200-mal bis ca. 2000-mal im Lebenszyklus des Verdampfers auftreten. Ferner steht der Träger im Kontakt zu der Flüssigkeit und/oder dem Aerosol bzw. Dampf und muss deshalb insbesondere bei den während der Verdampfung auftretenden Temperaturen lebensmitteltauglich bzw. biokompatibel sein, was durch das keramische Substrat begünstigt ist.

In einer bevorzugten Ausführungsform ist der Träger aus wenigstens ein oder zwei Schalen, vorzugsweise aus Kunststoff, gebildet. Ein einschaliger oder zweischaliger Träger kann einer besonders einfachen und kosteneffektiven Ausführungsform zuträglich sein. Ein Träger aus Kunststoff kann besonders kosteneffektiv hergestellt werden, beispielsweise durch Spritzgießen, und eine vorteilhafte Geometrie insbesondere zur Luftführung und/oder zur Halterung des Trägers in dem Grundteil aufweisen.

Erfindungsgemäß ist eine sich durch die Durchgangsöffnung erstreckende Dochtstruktur vorgesehen, um eine kapillare Flüssigkeitsleitung aus dem externen Teil zum Verdampfer durch die Flüssigkeitsöffnung und die Durchgangsöffnung zu ermöglichen. Damit ist eine von der Orientierung des Verdampfereinsatzes unabhängige und blasenfreie Flüssigkeitsversorgung des Verdampfers möglich.

Vorteilhaft weist der Verdampfereinsatz eine Längsachse auf, und eine Auslassseite des Verdampfers ist parallel zur Längsachse ausgerichtet, um eine effektive Anordnung des Verdampfers in dem Grundteil und eine vorteilhafte Zugabe der verdampften Flüssigkeit zu dem Luftstrom zu ermöglichen.

Vorzugsweise umfasst der Träger ein Wärmeleitungselement und/oder ein thermisches Isolierelement. Ein thermisches Isolierelement kann vorgesehen sein, um beim Erhitzen des Verdampfers die Erzeugung von Verlustwärme und/oder eine Wärmeableitung in die Umgebung des Verdampfers möglichst gering zu halten. Durch ein Wärmeleitungselement kann die Abwärme des Verdampfers dazu genutzt werden, beispielsweise die Flüssigkeit bei der Zuführung zu dem Verdampfer vorzuwärmen. Auf diese Weise kann die Effizienz des Verdampfungsprozesses gesteigert werden, da die Liquidförderung durch die thermisch bedingte Änderung der Viskosität begünstigt wird und weniger Energie zum Verdampfen benötigt wird. Das Wärmeleitungselement kann dazu eingerichtet sein, die am Verdampfer erzeugte Wärme zu nutzen, um eine vor Erwärmung der Zuluft stromaufwärts des Verdampfers und/oder einen Nacherwärmung des Aerosols bzw. Dampfes stromabwärts des Verdampfers zu bewerkstelligen. Das Wärmeleitungselement und/oder das thermische Isolierelement kann so eingesetzt werden, dass alle Werkstoffe des Verdampfereinsatzes in jedem Betriebszustand innerhalb ihrer spezifizierten maximalen Temperatur bleiben. Das Wärmeleitelement kann vorteilhaft aus einem metallischen Werkstoff bestehen. Das Isolierelement kann vorteilhaft aus einem keramischen und/oder Verbundwerkstoff bestehen.

Gemäß einem Aspekt der Erfindung umfasst der Träger einen Strömungs- und/oder Füllstandssensor, bevorzugt weiterhin einen Temperatursensor, um möglichst viele Funktionen in den Träger und somit in den Verdampfereinsatz zu integrieren. Der Strömungssensor kann den Luftstrom in dem Luftkanal und/oder in einem Nebenluftkanal messen. Der Temperatursensor kann die Temperatur des Zuluftstroms, des Verdampfers und/oder des erzeugten Dampfes bzw. Aerosols messen. Der Füllstandsensor kann so eingerichtet sein, dass der Füllstand eines externen Flüssigkeitsspeicher in dem der Verdampfereinsatz eingesetzt ist gemessen werden kann. Für einen oder jeden der Sensoren kann wenigstens eine elektrische Leitung in dem Träger integriert und/oder auf dem Träger aufgebracht werden. Die elektrische Kontaktierung kann für einen oder jeden der Sensoren einen Kontakt zum Auslesen des oder der jeweiligen Sensoren durch ein externes Teil aufweisen.

Gemäß einem Aspekt der Erfindung umfasst der Träger eine elektronische Steuereinrichtung, um beispielsweise den Betrieb des Verdampfers und/oder die Datenübertragung zwischen dem Träger und somit der Verdampfereinheit und einem externen Teil zu steuern. Die elektronische Steuereinrichtung kann einen ID-Chip bzw. eine Kennung zur Identifizierung des Verdampfereinsatzes von einem externen Teil umfassen.

Das Grundteil ist vorzugsweise patronen- oder hülsenförmig. Die Patronenform beziehungsweise vorteilhaft Hülsenform des Verdampfereinsatzes ist platzsparend und weist vorteilhaft eine neutrale Formgebung auf, was weitestgehende Freiheit bei der Gestaltung eines den Verdampfereinsatz umgebenden externen Teils und/oder eines Inhalators lässt. Aufgrund der Patronen- bzw. Hülsenform des Verdampfereinsatzes ist vorteilhaft eine vielseitige Einbringungsmöglichkeit in unterschiedliche Tanks ermöglicht. Die Integration der Luft- und/oder Aerosolführung durch den Luftstromkanal innerhalb des Verdampfereinsatzes und ein vorteilhaftes Anbindungskonzept durch die Flüssigkeitsöffnung an unterschiedliche Tanks liefern eine standardisierte Aerosolqualität und gegebenenfalls eine verbesserte Dichtigkeit der Produkte, beispielsweise der Inhalatoren.

Bevorzugt weist der Verdampfereinsatz beziehungsweise das Grundteil entlang des Strömungskanals einen abschnittsweise gleichbleibenden und/oder sich monoton verjüngenden Querschnitt auf, um die Montage des Verdampfereinsatzes zu begünstigen. Der Querschnitt kann abschnittsweise oder vollumfänglich kreisrund, mehr- oder vieleckig und/oder oval sein. Ein wenigstens abschnittsweise unrunder Querschnitt erlaubt eine verdrehsichere Montage des Verdampfereinsatzes in einem externen Teil.

Vorzugsweise ist der Verdampfereinsatz in einem externen Teil, insbesondere in einem Flüssigkeitsspeicher, anordenbar, insbesondere in dieses einsetzbar oder einschiebbar, um eine einfache und effektive Montage zu erlauben. Der Verdampfereinsatz ist vorzugsweise ein Einsatz zum Anordnen, Einsetzen, beispielsweise Einschieben, Eindrehen und/oder Einschrauben in den Flüssigkeitsspeicher eines Inhalators.

Bevorzugt umfasst der Verdampfereinsatz mindestens ein Halte- und/oder Befestigungselement zur Halterung und/oder Befestigung des Verdampfereinsatzes in dem externen Teil, um ein unbeabsichtigtes Lösen des Verdampfereinsatzes in dem externen Teil zu verhindern. Der Verdampfereinsatz kann auch ein Gegenstück zu einem Halte- und/oder Befestigungselement in dem externen Teil vorsehen. Die Befestigung des Verdampfereinsatzes in dem externen Teil kann lösbar oder unlösbar sein.

Bevorzugt ist der Verdampfereinsatz in das externe Teil verdrehsicher einbaubar, um zu gewährleisten, dass die Flüssigkeitsöffnung beispielsweise einer entsprechenden Zuführöffnung im externen Teil zugeordnet ist und/oder die elektrische Kontaktierung in eine Wirkverbindung mit elektrischen Kontakten eines Basisteils des Inhalators in Verbindung gebracht werden können, um beispielsweise eine Versorgung mit elektrischer Energie aus einem Energiespeicher herzustellen. Vorzugsweise umfasst der Verdampfereinsatz ein Verdrehsicherungselement an der Mantelseite des Grundteils, wie beispielsweise Rastelement, Nase, Nut und/oder Aussparung.

Es ist von Vorteil, wenn in dem Grundteil mindestens eine luftdurchlässige Be- und/oder Entlüftungseinrichtung zur Be- und/oder Entlüftung eines Flüssigkeitsspeichers vorgesehen ist, um ungewollte Druckunterschiede zwischen dem Flüssigkeitsspeicher und dem Luftstromkanal zu vermeiden. Durch die Verdampfung von aus dem Flüssigkeitsspeicher zugeführter Flüssigkeit entsteht in dem Flüssigkeitsspeicher ein Unterdruck bzw. am Verdampfer ein Druckgefälle, das dazu führen kann, dass Liquid vom Verdampfer weggedrückt wird und dieser damit trocken fällt. Ein Druckgefälle am Verdampfer kann auch dadurch entstehen, dass das Gerät auf große Höhen befördert wird, zum Beispiel beim Transport in einem Flugzeug, wobei der verringerter Außendruck zu einem unkontrollierten Austritt von Liquid aus dem Flüssigkeitsspeicher führen kann. Ein Unterdruck kann auch durch das Saugen des Konsumenten an einem Mundende des Inhalators entstehen, während der Verdampfer nicht beheizt ist. Dies kann zu Liquidaustritt durch den Verdampfer führen. Dies kann durch eine Be- und/oder Entlüftung des Flüssigkeitsspeichers über beispielsweise die Mantelseite des Verdampfereinsatzes verhindert werden, da dann gleichzeitig am Flüssigkeitsspeicher und damit an der Rückseite beziehungsweise der dem Flüssigkeitsspeicher zugewandten Seite des Verdampfers ein Unterdruck anliegt. Die Be- und/oder Entlüftung kann so ausgelegt sein, dass unabhängig von der räumlichen Orientierung des Verdampfereinsatzes gewährleistet ist, dass nur Luft durch die Be- und/oder Entlüftungseinrichtung treten kann und der Durchtritt von Liquid unterbunden ist.

Bevorzugt weist der Verdampfereinsatz an der Mantelseite des Grundteils mindestens ein Halteelement, beispielsweise einen Dorn, zur Halterung eines Flüssigkeitszuführelementes auf, um die Flüssigkeitsöffnung unabhängig von der Orientierung des Verdampfereinsatzes mit Flüssigkeit versorgen zu können. Das Flüssigkeitszuführelement, beispielsweise ein kapillares Element, ist dazu eingerichtet, Flüssigkeit zu speichern und beispielsweise einer Dochtstruktur zuzuführen. Damit kann auch bei kurzzeitigem Betrieb eines Inhalators mit dem Verdampfereinsatz über Kopf ausreichend Liquid bereitgestellt werden, um den Konsum des Inhalators zu ermöglichen.

In einer weiteren Ausgestaltung umfasst eine Verdampfer-Tank-Einheit beziehungsweise eine Verbrauchseinheit für ein elektronisches Zigarettenprodukt einen zuvor beschriebenen, erfindungsgemäßen Verdampfereinsatz und einen Flüssigkeitsspeicher, wobei der Verdampfereinsatz in den Flüssigkeitsspeicher eingesetzt ist. Der Flüssigkeitsspeicher kann dazu eine vorteilhafte standardisierte Flüssigkeitsschnittstelle umfassen, mit der die dazu korrespondierende Flüssigkeitsöffnung des Verdampfereinsatzes flüssigkeitsleitend und leckagefrei verbunden werden kann. Beispielsweise kann die so entstehende Flüssigkeitsschnittstelle eine Dichtung aufweisen. Der Flüssigkeitsspeicher kann standardisierte Halteelemente zum verschiebefesten Halten des Verdampfereinsatzes aufweisen. Die äußere Geometrie des Flüssigkeitsspeichers kann somit anwendungsspezifisch und unabhängig vom Verdampfereinsatz gestaltet sein.

Der Flüssigkeitsspeicher weist vorzugsweise standardisiert eine Einsetzöffnung, eine Ausgangsöffnung und/oder einen der äußeren Form des Verdampfereinsatzes entsprechend geformten Montagekanal mit einer Einsetzöffnung auf, in welche der Verdampfereinsatz anordenbar und/oder einschiebbar ist.

Nach einer weiteren Ausgestaltung umfasst ein elektronisches Zigarettenprodukt eine Verdampfer-Tank-Einheit und ein Basisteil, wobei die Verdampfer-Tank-Einheit einen standardisierten Verdampfereinsatz und/oder einen Verdampfereinsatz mit einem standardisierten Träger aufweist. Die Standardisierung kann insbesondere durch die Geometrie und/oder der elektrischen Kontaktierung des Trägers, des Verdampfereinsatzes und/oder des Flüssigkeitsspeichers gegeben sein.

Ein Verfahren zur Herstellung des Verdampfereinsatzes kann die folgenden Schritte umfassen: Bereitstellen des Grundteils und des Trägers, Einsetzen und/oder Einschieben des Trägers in das Grundteil, um ein möglichst einfaches und effektives Verfahren zur Herstellung des Verdampfereinsatzes bereitzustellen. Der Träger wird beispielsweise in das Innere des Grundteils vorteilhaft eingeschoben, um sich an einer Wandung des Grundteils stabil abstützen zu können.

Ein Verfahren zur Herstellung der Verdampfer-Tank-Einheit könnte die folgenden Schritte umfassen: Bereitstellen des Verdampfereinsatzes und eines Flüssigkeitsspeichers, Einsetzen und/oder Einschieben des Verdampfereinsatzes den Flüssigkeitsspeicher, um ein möglichst einfaches und effektives Verfahren zur Herstellung der Verdampfer-Tank-Einheit bereitzustellen. Der Verdampfereinsatz kann insbesondere in eine Einsetzöffnung des Flüssigkeitsspeichers eingesetzt werden. Dabei kann der Verdampfereinsatz, der länger als der Flüssigkeitstank sein kann, auch teilweise beispielsweise mit einem Luft-Dampf-Schlot aus der anderen Seite des Flüssigkeitsspeichers hinausragen und ggf. nach dem Einsetzen gekürzt werden.

Die Erfindung wird im Folgenden anhand bevorzugter Ausführungsformen unter Bezugnahme auf die beigefügten Figuren erläutert. Dabei zeigt
- Fig. 1: eine schematische Darstellung eines elektronischen Zigarettenproduktes;
- Fig. 2: einen Schnitt durch einen Verdampfereinsatz;
- Fig. 3: eine Explosionsdarstellung eines Trägers;
- Fig. 4: einen Schnitt durch einen Träger;
- Fig. 5: einen Schnitt durch einen Verdampfereinsatz;
- Fign. 6, 7: jeweils eine Darstellung der Herstellung eines Verdampfereinsatzes;
- Fig. 8: eine perspektivische Ansicht eines Verdampfers mit einem Flüssigkeitsspeicher; und
- Fig. 9: einen Längsschnitt durch eine Verdampfer-Tank-Einheit.

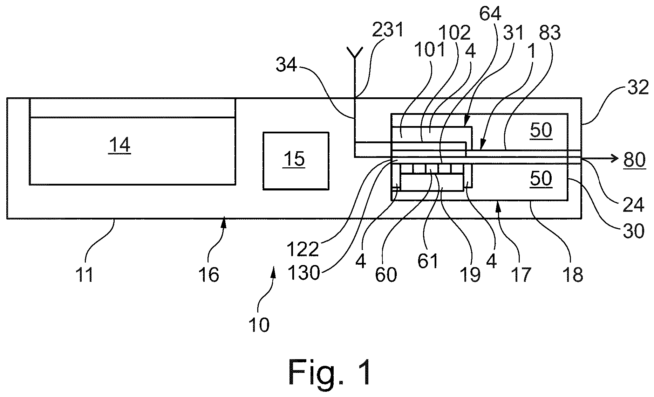

Figur 1 zeigt schematisch ein elektronisches Zigarettenprodukt 10 beziehungsweise einen Inhalator. Das elektronische Zigarettenprodukt 10 umfasst ein Gehäuse 11, in dem ein Luftstromkanal 30 zwischen mindestens einer Lufteinlassöffnung 231 und einer Luftauslassöffnung 24 an einem Mundende 32 des Zigarettenprodukts 10 vorgesehen ist. Das Mundende 32 des Zigarettenprodukts 10 bezeichnet dabei das Ende, an dem der Konsument zwecks Inhalation zieht und dadurch das Zigarettenprodukt 10 mit einem Unterdruck beaufschlagt und einen Luftstrom 34 in dem Luftstromkanal 30 erzeugt.

Das Zigarettenprodukt 10 besteht vorteilhaft aus einem Basisteil 16 und einer Verbrauchseinheit beziehungsweise Verdampfer-Tank-Einheit 17, die die Verdampfervorrichtung 1 und den Flüssigkeitsspeicher 18 umfasst und insbesondere in Form einer auswechselbaren Kartusche ausgebildet ist. Die durch die Einlassöffnung 231 angesaugte Luft wird in dem Luftstromkanal 30 zu der, oder durch die mindestens eine Verdampfervorrichtung 1 geleitet. Die Verdampfervorrichtung 1 ist mit dem Flüssigkeitsspeicher 18 verbunden oder verbindbar, in dem mindestens eine Flüssigkeit 50 gespeichert ist.

Die Verdampfervorrichtung 1 verdampft Flüssigkeit 50, die der Verdampfervorrichtung 1 aus dem Flüssigkeitsspeicher 18 vorteilhaft von einem Docht beziehungsweise einer Dochtstruktur 19 mittels Kapillarkräften zugeführt wird, und gibt die verdampfte Flüssigkeit als Aerosol/Dampf an einer Auslassseite 64 in den Luftstrom 34 zu.

An einer Einlassseite 61 des Heizkörpers 60 ist vorteilhaft die poröse und/oder kapillare, flüssigkeitsleitende Dochtstruktur 19 angeordnet, wie schematisch in Figur 1 gezeigt. Es können eine Flüssigkeitsschnittstelle und/oder mehrere Flüssigkeitsleitungen zwischen Flüssigkeitsspeicher 18 und Dochtstruktur 19 vorgesehen sein. Der Flüssigkeitsspeicher 18 kann daher auch beabstandet von der Dochtstruktur 19 angeordnet sein. Die Dochtstruktur 19 kontaktiert die Einlassseite 61 des Heizkörpers 60 vorteilhaft flächig und deckt sämtliche Flüssigkeitskanäle 62 des Heizkörpers 60 einlassseitig ab. An der dem Heizkörper 60 gegenüberliegenden Seite ist die Dochtstruktur flüssigkeitsleitend mit dem Flüssigkeitsspeicher 18 verbunden. Der Flüssigkeitsspeicher 18 kann in seinen Abmessungen größer als die Dochtstruktur 19 sein. Die Dochtstruktur 19 kann beispielsweise in eine Öffnung eines Gehäuses des Flüssigkeitsspeichers 18 eingesetzt sein. Es kann auch eine Mehrzahl von Verdampfervorrichtungen 1 einem Flüssigkeitsspeicher 18 zugeordnet sein. Die Dochtstruktur 19 kann generell einteilig oder mehrteilig sein.

Die Dochtstruktur 19 besteht aus porösem und/oder kapillarem Material, das aufgrund von Kapillarkräften in der Lage ist, von dem Heizkörper 60 verdampfte Flüssigkeit in ausreichender Menge von dem Flüssigkeitsspeicher 18 zu dem Heizkörper 60 passiv nachzufördern, um ein Leerlaufen der Flüssigkeitskanäle 62 und sich daraus ergebende Probleme zu verhindern.

Die Dochtstruktur 19 besteht vorteilhaft aus einem elektrisch nichtleitenden Material, um eine unerwünschte Erwärmung von Flüssigkeit in der Dochtstruktur 19 durch Stromfluss zu vermeiden. Die Dochtstruktur 19 weist vorteilhaft eine geringe thermische Leitfähigkeit auf. Die Dochtstruktur 19 besteht vorteilhaft aus einem oder mehreren der Materialien Baumwolle, Cellulose, Acetat, Glasfasergewebe, Glasfaserkeramik, Sinterkeramik, keramisches Papier, Alumosilikat-Papier, Metallschaum, Metallschwamm, einem anderen hitzebeständigen, porösen und/oder kapillaren Material mit geeigneter Förderrate, oder einem Verbund von zwei oder mehr der vorgenannter Materialien. In einer vorteilhaften praktischen Ausführungsform kann die Dochtstruktur 19 mindestens ein Keramikfaserpapier und/oder eine poröse Keramik umfassen. Das Volumen der Dochtstruktur 19 liegt vorzugsweise im Bereich zwischen 1 mm^3 und 10 mm^3, weiter vorzugsweise im Bereich zwischen 2 mm^3 und 8 mm^3, noch weiter vorzugsweise im Bereich zwischen 3 mm^3 und 7 mm^3 und beträgt beispielsweise 5 mm^3.

Falls die Dochtstruktur 19 aus einem elektrisch und/oder thermisch leitenden Material besteht, ist zwischen der Dochtstruktur 19 und dem Heizkörper 60 vorteilhaft eine Isolierschicht aus einem elektrisch und/oder thermisch isolierenden Material, beispielsweise Glas, Keramik oder Kunststoff, mit sich durch die Isolierschicht erstreckenden, mit den Flüssigkeitskanälen 62 korrespondierenden Öffnungen vorgesehen.

Ein vorteilhaftes Volumen des Flüssigkeitsspeichers 18 liegt im Bereich zwischen 0,1 ml und 5 ml, vorzugsweise zwischen 0,5 ml und 3 ml, weiter vorzugsweise zwischen 0,7 ml und 2 ml oder 1,5 ml.

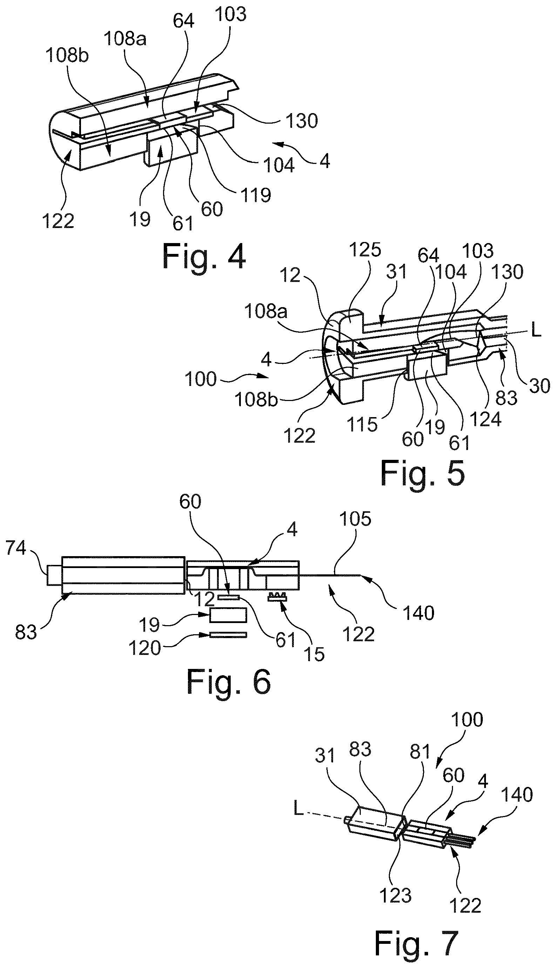

Die elektronische Zigarette 10 umfasst des Weiteren einen elektrischen Energiespeicher 14 und eine elektronische Steuerungsvorrichtung 15. Der Energiespeicher 14 ist in der Regel in dem Basisteil 16 angeordnet und kann insbesondere eine elektrochemische Einweg-Batterie oder ein wiederaufladbarer elektrochemischer Akku, beispielsweise ein Lithium-Ionen-Akku, sein. Die Verdampfer-Tank-Einheit 17 ist zwischen dem Energiespeicher 14 und dem Mundende 32 angeordnet. Die elektronische Steuerungsvorrichtung 15 umfasst mindestens eine digitale Datenverarbeitungseinrichtung, insbesondere Mikroprozessor und/oder Microcontroller, in dem Basisteil 16 (wie in Figur 1 gezeigt) und/oder in der Verdampfer-Tank-Einheit 17 beziehungsweise einem Verdampfereinsatz 100 (wie in Figuren 6 und 7 gezeigt).

In dem Gehäuse 11 ist vorteilhaft ein Sensor, beispielsweise ein Drucksensor oder ein Druck- oder Strömungsschalter, angeordnet, wobei die Steuerungsvorrichtung 15 auf der Grundlage eines von dem Sensor ausgegebenen Sensorsignals feststellen kann, dass ein Konsument am Mundende 32 des Zigarettenprodukts 10 zieht, um zu inhalieren. In diesem Fall steuert die Steuerungsvorrichtung 15 die Verdampfervorrichtung 1 an, um Flüssigkeit 50 aus dem Flüssigkeitsspeicher 18 als Aerosol/Dampf in den Luftstrom 34 zuzugeben.

Die Verdampfervorrichtung 1 beziehungsweise der mindestens eine Verdampfer 60 ist in einem dem Mundende 32 abgewandten Teil der Verdampfer-Tank-Einheit 17 angeordnet. Damit ist eine effektive elektrische Kopplung und Ansteuerung der Verdampfervorrichtung 1 möglich. Der Luftstrom 34 führt vorteilhaft durch einen axial durch den Flüssigkeitsspeicher 18 laufenden Luftstromkanal 30 zu der Luftauslassöffnung 24.

Die in dem Flüssigkeitsspeicher 18 gespeicherte, zu dosierende Flüssigkeit 50 ist beispielsweise eine Mischung aus 1,2-Propylenglykol, Glycerin, Wasser, mindestens einem Aroma (Flavour) und/oder mindestens einem Wirkstoff, insbesondere Nikotin. Die angegebenen Bestandteile der Flüssigkeit 50 sind jedoch nicht zwingend. Insbesondere kann auf Aroma- und/oder Wirkstoffe, insbesondere Nikotin, verzichtet werden.

Die Verdampfer-Tank-Einheit bzw. Kartusche 17 oder das Basisteil 16 umfasst vorteilhaft einen nichtflüchtigen Datenspeicher zum Speichern von die Verdampfer-Tank-Einheit bzw. Kartusche 17 betreffender Information bzw. Parameter. Der Datenspeicher kann Teil der elektronischen Steuerungsvorrichtung 15 sein. In dem Datenspeicher ist vorteilhaft Information zur Zusammensetzung der in dem Flüssigkeitsspeicher 18 gespeicherten Flüssigkeit, Information zum Prozessprofil, insbesondere Leistungs-/Temperatursteuerung; Daten zur Zustandsüberwachung bzw. Systemprüfung, beispielsweise Dichtigkeitsprüfung; Daten betreffend Kopierschutz und Fälschungssicherheit, eine ID zur eindeutigen Kennzeichnung der Verdampfer-Tank-Einheit bzw. Kartusche 17, Seriennummer, Herstelldatum und/oder Ablaufdatum, und/oder Zugzahl (Anzahl der Inhalationszüge durch den Konsumenten) bzw. der Nutzungszeit gespeichert. Der Datenspeicher ist vorteilhaft elektrisch mit der Steuereinrichtung 15 verbunden oder verbindbar.

In dem Inhalator 10 und/oder in einem externen Speicher, der in geeigneter und an sich bekannter Weise, zumindest zeitweilig, kommunikationstechnisch mit dem Inhalator 10 verbunden werden kann, könnten auch nutzerbezogene Daten, insbesondere über das Rauchverhalten, gespeichert und vorzugsweise auch zur Steuerung und Regelung des Inhalators genutzt werden.

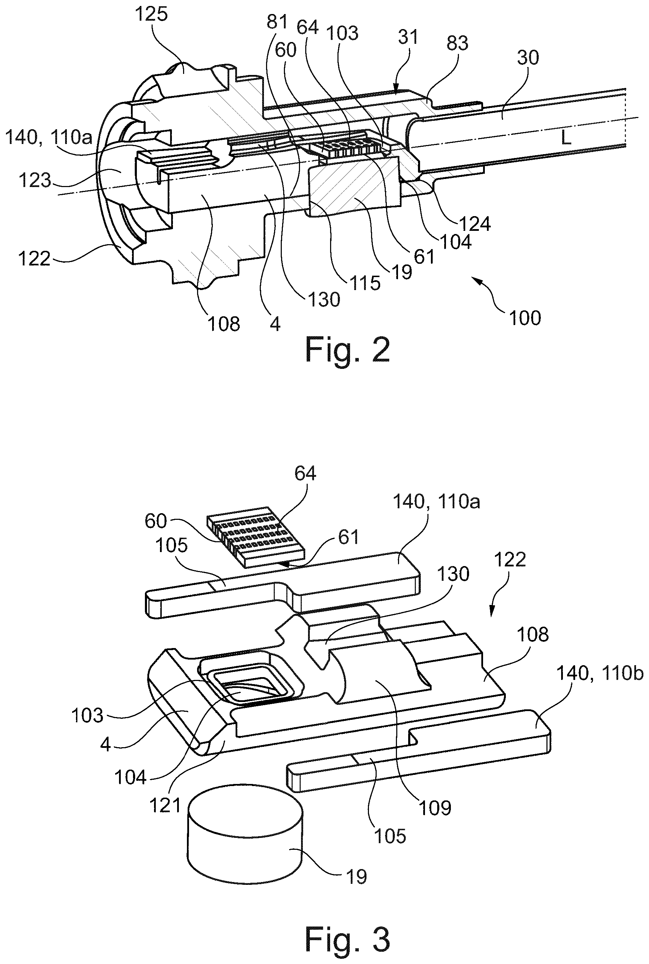

Ein Grundteil 83 und ein Träger 4 bilden einen Verdampfereinsatz 100, siehe Figur 2, der flüssigkeitsdicht ist und keine Flüssigkeit 50 in den Innenraum des Verdampfereinsatzes 100 vordringen lässt, um einen ungewollten Austritt von Flüssigkeit 50 aus dem Luftstromkanal 30 und/oder der Verdampfer-Tank-Einheit 17 zu verhindern. Die Abdichtung des Verdampfereinsatzes 100 ist so ausgebildet, dass Flüssigkeit 50 nur den Weg durch die Dochtstruktur 19 und anschließend durch den Verdampfer 60 nehmen kann und im verdampften Zustand dem Luftstrom 34 zugegeben wird. Für die Flüssigkeitsdichtigkeit können nach beziehungsweise während der Montage der Verdampfer-Tank-Einheit 17 Klebungen, Presspassungen, zusätzliche Dichtmaterialien wie eine Dichtung 120 und/oder Verschweißungen verwendet werden. Zur Entlüftung des Flüssigkeitsspeichers 18 kann der Verdampfereinsatz 100 eine semipermeable Membran aufweisen, die Luft in den Flüssigkeitsspeicher 18 hineintreten und keine Flüssigkeit 50 aus den Flüssigkeitsspeicher 18 austreten lässt.

Der Verdampfereinsatzes 100 umfasst das Grundteil 83 mit einer um den Luftstromkanal 30 herum angeordneten Mantelseite 31, siehe Figur 2. Durch den Verdampfereinsatz 100 verläuft der Luftstromkanal 30. Der Luftstromkanal 30 verläuft im Wesentlichen entlang einer Längsachse L, wobei das Grundteil 83 entlang der Längsachse L die größte Ausdehnung aufweist.

Der Verdampfereinsatz 100 weist einen mit Bezug zu Figur 3 erläuterten Träger 4 auf. Der Träger 4 ist an einer Wandung 81 des Grundteils 83 abgestützt, wobei die Mantelseite 31 die Wandung 81 bildet, an der sich der Träger 4 mit der Grundplatte 121 und einer Auswölbung 109 abstützt. Dafür weist der Träger 4 wenigstens teilweise einen Querschnitt auf, der dem Querschnitt der Wandung 81 entspricht.

Die Auswölbung 109 weist einen teilweise runden Querschnitt auf, der dem Innenquerschnitt einer Wandung 81 des Grundteils 83 entspricht. Die Abmessung der im wesentlichen plattenförmigen Grundplatte 121 weist eine Kantenlänge auf, die gleich dem Innenquerschnitt des Grundteils 83 ist, um sicher in dem Grundteil 83 gehalten werden zu können.

Innerhalb der Auswölbung bildet der Träger 4 einen Luftkanal 130 zum Führen des Luftstroms 34 aus. Die Auswölbung 109 kann zudem dazu dienen, den Träger 4 bzw. die Schale 108 in dem Grundteil 83 des Verdampfereinsatzes 100 halten zu können, (siehe Figuren 2 und 5).

Der Verdampfereinsatz 100 weist den vom Träger 4 gehaltenen Verdampfer 60 auf. Die Auslassseite 64 ist dem Inneren des Verdampfereinsatzes 100 bzw. dem Luftkanal 130 zugewandt. Der Verdampfer 60 ist so angeordnet, dass die vorteilhaft planare Auslassseite 64 parallel zur Längsachse L angeordnet ist, um vorteilhaft von dem Luftstrom 34 überströmt werden zu können. In anderen Ausführungsformen kann die Auslassseite 64 auch gekrümmt und/oder zur Längsachse geneigt angeordnet sein, um die Überströmung der Auslassseite 64 anzupassen.

Der Luftkanal 130 geht stromabwärts des Verdampfers 60 in den Luftstromkanal 30 über. Der Luftkanal 130 kann als der vom Träger 4 gebildete Strömungsabschnitt des Luftstromkanals 130 aufgefasst werden.

Der Aufbau des Trägers 4, insbesondere die Anordnung des Luftkanals 130 und der Auslassseite 64 des Verdampfers 60 gewährleisten in der Aerosolphase eine Strömungsgeschwindigkeit, die verhindert, dass Tröpfchen im Luftstrom 34 an den Wandungen 31 des Grundteils 83, an dem Trägers 4 und/oder an Luftstrom-Umlenkpunkten durch Anprallen gesammelt werden und so Flüssigkeit 50 aus dem Inhalator 10 austritt. Der Luftdruck am Verdampfer 60, insbesondere ein Unterdruck, der Zugwiderstand und/oder Verwirbelungen im Luftstrom 34 können über den Querschnitt des Luftstromkanals 30 und/oder gezielte Verengungen gesteuert werden. Dies kann den Luftstrom 34 beim Auftreffen auf den Verdampfer 60 und die Aerosolqualität, wie beispielsweise die Durchmischung und/oder Tröpfchengröße beeinflussen.

Zusätzliche Kanäle, insbesondere wenigstens ein einen Nebenluftstrom 102 führenden Nebenluftkanal 101, die stromabwärts vom Verdampfer 60 auf den Luftkanal 130 treffen, können für Durchmischung des Gas-/Aerosol-Gemisches mit Frischluft sorgen und/oder Prozesse der Nachbehandlung und/oder der Rekondensation regeln (siehe Figur 1). Ein optional einstellbarer Querschnitt des Luftstromkanals 30 und/oder eines Nebenluftkanals 101 ermöglicht dem Nutzer die Einstellung eines individuellen Zugwiderstandes und/oder einer individuellen Aerosol-Qualität.

Der Träger 4 bzw. die Schale 108 weist eine Durchgangsöffnung 104 auf, siehe Figuren 2 und 3. Um die Durchgangsöffnung 104 ist ein keramisches Substrat 103 zur Halterung eines Verdampfers 60 angeordnet. Das keramische Substrat 103 kann aus einer Niedertemperatur-Einbrand-Keramik (LTCC) bestehen. Das keramische Substrat 103 kann der thermischen Entkopplung von dem Verdampfer 60 und der Grundplatte 103 dienen und ist hitzebeständig und thermisch stabil.

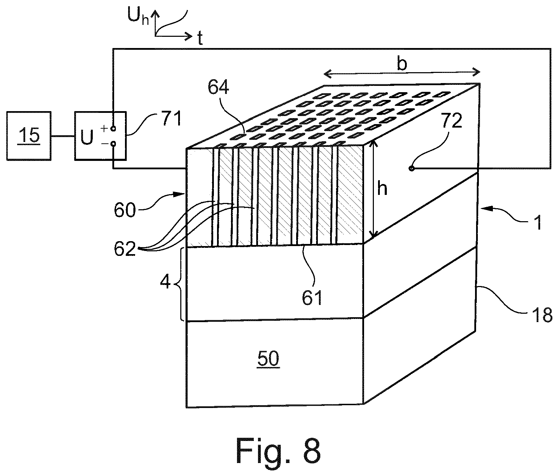

Die Einlassseite 61 des Verdampfers 60 ist vorteilhaft flüssigkeitsleitend mit einer Dochtstruktur 19 verbunden. Die Dochtstruktur 19 wird mit Bezug zu Figuren 1 und 8 erläutert und erstreckt sich vorteilhaft durch die Durchgangsöffnung 104. Die Durchgangsöffnung 104 ist vorteilhaft so bemessen, dass sich die Dochtstruktur 19 durch die Durchgangsöffnung 104 erstrecken kann. Die Dochtstruktur 19 kann auch in der Durchgangsöffnung 104 abgestützt und gehalten sein. Alternativ kann sich der Verdampfer 60 durch die Durchgangsöffnung 104 erstrecken, um von der Dochtstruktur 19 einlassseitig flüssigkeitsleitend kontaktiert zu werden. In dieser Ausführungsform kann die Dochtstruktur 19 beispielsweise einen größeren Querschnitt als die Durchgangsöffnung 104 aufweisen.

An der Mantelseite 31 des Grundteils 83 ist eine Flüssigkeitsöffnung 115 zum Zuführen von verdampfbarer Flüssigkeit 50 von außen in den Verdampfereinsatz 100 zu dem Verdampfer 60 angeordnet, siehe Figur 2. Die Durchgangsöffnung 104, die zur Flüssigkeitsversorgung des Verdampfers 60 mit der Flüssigkeitsöffnung 115 korrespondiert, und die Flüssigkeitsöffnung 115 kann ein externes Teil, beispielsweise einen Flüssigkeitsspeicher 18 mit dem Verdampfer 60 flüssigkeitsleitend verbinden. Die Durchgangsöffnung 104 ist an dem Träger 4 so angeordnet, dass ihre Größe und/oder Position im montierten Zustand einen Transport von Flüssigkeit 50 durch die Flüssigkeitsöffnung 115 erlaubt. Beispielsweise können die Flüssigkeitsöffnung 115 und die Durchgangsöffnung 104 eine ähnliche oder die gleiche Abmessung aufweisen. In dieser Ausführungsform weist die Durchgangsöffnung 104 die gleiche Größe wie die Flüssigkeitsöffnung 115 auf. Im montierten Zustand des Verdampfereinsatzes 100 ist die Durchgangsöffnung 104 mit der Flüssigkeitsöffnung 115 wenigstens teilweise überdeckt angeordnet.

Durch die Flüssigkeitsöffnung 115 und/oder die Durchgangsöffnung 104 kann sich die Dochtstruktur 19 erstrecken. Die Dochtstruktur 19 kann einerseits mit der Einlassseite 61 des Verdampfers 60 flüssigkeitsleitend verbunden sein und andererseits zur Aufnahme von Flüssigkeit 50 in einem externen Teil 18 angeordnet sein. Dafür kann die Dochtstruktur 19 bündig mit der Mantelseite 31 abschließen oder die Mantelseite 31 überragen. Die Dochtstruktur 19 kann von der Durchgangsöffnung 104 und/oder der Flüssigkeitsöffnung 115 gehalten sein.

Das Grundteil 83 weist vorteilhaft einen Montageschacht 123 zum Einsetzen beziehungsweise Einschieben des Trägers 4 auf. Der Montageschacht 123 ist vorteilhaft durch die Wandung 81 begrenzt. Der Träger 4 kann so weit in den Montageschacht 123 eingeschoben werden, bis der Montageschacht 123 beispielsweise an einer Verengung 124 beziehungsweise einem Anschlag endet. Vorteilhaft ist die Länge des Montageschachts 123 wenigstens so groß wie die Länge des Trägers 4, so dass der Träger 4 bündig mit dem sich stromaufwärts des Verdampfers 60 befindlichen Ende 122 abschließen kann und/oder gegenüber dem Ende 122 entlang der Längsachse L in das Grundteil 83 verschoben ist.

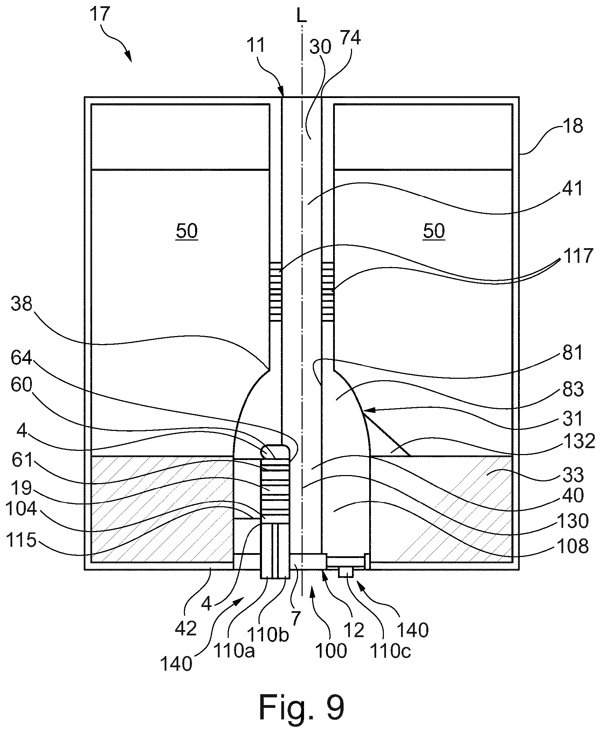

Zur vorteilhaften Handhabbarkeit des Verdampfereinsatzes 100 weist das Grundteil 83 an dem sich stromaufwärts des Verdampfers 60 befindliche Ende 122 einen Kragen 125 und/oder eine Bodenplatte 7 auf, siehe Figur 9, der bzw. die sich axial von der Mantelseite 31 des Grundteils 83 erstreckt.

Der Träger 4 für den Verdampfereinsatz 100 umfasst eine Schale 108 mit einer im Wesentlichen plattenförmigen Grundfläche 121, siehe Figur 3. Die Schale 108 ist beispielsweise aus einem Kunststoff gefertigt, insbesondere einem Hochtemperaturkunststoff wie PEEK, der bei den bei der Verdampfung auftretenden Temperaturen von bis 300 °C chemisch und mechanisch stabil ist.

Die Ausformung beziehungsweise Geometrie des Trägers 4 ist vorteilhaft so angepasst, dass die nötige Luftführung integriert ist. Entsprechende Aussparungen und/oder Ausformungen, beispielsweise die Auswölbung 109, können sowohl die Zuluft zum Verdampfer 60, die Überströmung des Verdampfers 60 und/oder die Führung des Aerosols beziehungsweise Dampfes lenken. So kann die Verdampfung optimiert werden, indem an der Auslassseite 64 des Verdampfers 60 der Luftstrom 34 so eingestellt wird, dass eine gewünschte Menge an Tröpfchen mit entsprechender Größe dem Luftstrom 34 zugegeben wird.

Der Träger 4 bzw. die Schale 108 weist für die elektrische Versorgung des Verdampfereinsatzes 100 und/oder zum Empfangen von Steuersignalen für den Verdampfer 60 eine elektrische Kontaktierung 140 auf. Die elektrische Kontaktierung 140 umfasst hier zwei elektrische Kontakte 110a, 110b. Die elektrischen Kontakte 110a, 110b sind über elektrische Leitungen 105 von einem vorteilhaft stromaufwärts des Verdampfer 60 befindlichen Ende 122 des Trägers 4 mit dem Verdampfer 60 zur Versorgung des Verdampfers 60 mit elektrischer Energie verbunden. Das sich stromaufwärts des Verdampfers 60 befindliche Ende 122 ist das Ende, das durch die elektrische Kontaktierung 140 zur mechanischen und/oder elektrischen Verbindung mit einem externen Teil, beispielsweise einem Basisteil 16 des Inhalators 10, eingerichtet ist. Die elektrischen Leitungen 105 verlaufen vorzugsweise parallel zum Luftkanal 130.

Die elektrische Kontaktierung 140 umfasst in diesem Beispiel genau zwei elektrische Kontakte 110a, 110b, um eine denkbar einfache elektrische und somit auch mechanische Schnittstelle zwischen dem Verdampfereinsatz 100 und einem externen Teil bereitstellen zu können. Dabei können beispielsweise über Filter, Multiplexer und/oder Demultiplexer Signale so aufgetrennt werden, dass zwischen Heizimpulsen Sensordaten ausgelesen bzw. Signale an Sensoren übertragen werden können.

Der Träger 4 ist als standardisiertes Bauteil ausgeführt. Möglichst viele Funktionen des Verdampfereinsatzes 100 beispielsweise der elektrischen Steuerung und/oder der Luftführung, sind in den Träger 4 bzw. in von dem Träger 4 umfassten Komponenten integriert. In dem Verdampfereinsatz 100 sind möglichst viele Funktionen standardisiert integriert, insbesondere der Verdampfer 60, die Dochtstruktur 19, die elektrische Kontaktierungen 140, elektrische Leitungen 105, eine elektronische Steuerungsvorrichtung 15 beispielsweise umfassend eine ID, und eine definierte Luftführung, die beispielsweise durch den Luftkanal 130 im Bereich des Verdampfers 60 gebildet ist.

Figur 4 zeigt einen Schnitt durch einen Träger 4 für einen Verdampfereinsatz 100. Der Träger 4 ist aus zwei Schalen 108a, 108b gebildet. Die Schalen 108a, 108b sind so angeordnet, dass zwischen den Schalen 108a, 108b der Luftkanal 130 gebildet wird. Der Luftkanal 130 ist somit im Inneren des Trägers 4 angeordnet. Die Schalen 108a, 108b können als eine Unterschale 108b und eine Oberschale 108a angeordnet werden.

Eine der Schalen 108a, 108b weist beispielsweise die Durchgangsöffnung 104 zum Anordnen der Dochtstruktur 19 auf. Ein Vlies 119 kann zum verbesserten Flüssigkeitstransport zwischen der Einlassseite 61 und der Dochtstruktur 19 angeordnet sein. Beispielsweise kann das Vlies 119 thermisch und/oder chemisch besonders stabil sein und/oder eine andere Flüssigkeitsleit- und/oder Flüssig¬keits-speicher¬fähigkeit als die Dochtstruktur 19 aufweisen.

Eine der Schalen 108a, 108b weist vorteilhaft ein keramisches Substrat 103 zur Halterung des Verdampfers 60 auf. Vorteilhaft ist das keramisches Substrat 103 von der Schale 108b gehalten, die auch die Durchgangsöffnung 104 aufweist.

Der Außenquerschnitt des Trägers 4 beziehungsweise im montierten Zustand nach außen zeigende Abschnitte der Schalen 108a, 108b sind wenigstens teilweise gekrümmt, beispielsweise rund und/oder teilkreisförmig. Die Form des Trägers 4 bzw. der Schalen 108a, 108b ist vorteilhaft so ausgebildet, dass der Träger 4 wie in Figur 5 gezeigt innerhalb des Grundteils 83 angeordnet, abgestützt und gehalten werden kann.

Figur 5 zeigt einen Schnitt durch einen erfindungsgemäßen Verdampfereinsatz 100 mit dem mit Bezug zu Figur 4 erläuterten Träger 4 mit zwei Schalen 108a, 108b.

Der Träger 4 kann als vollständig montierte Einheit, umfassend die Schalen 108a, 108b in den Montageschacht 123 eigesetzt werden. Es ist jedoch auch möglich, dass zunächst eine Schale 108a und anschließen die andere Schale 108b in den Montageschacht 123 eingesetzt wird. Die Schalen 108a, 108b können beispielsweise miteinander verklemmt, verklebt, verschweißt und/oder verlötet sein.

Figuren 6 und 7 zeigen jeweils eine Darstellung der Herstellung eines Verdampfereinsatzes 100 wie in Figur 2 gezeigt, wobei Figur 6 einen Längsschnitt des Verdampfereinsatzes 100 und Figur 7 eine perspektivische Ansicht des Verdampfereinsatzes 100 zeigt. In Figuren 6 und 7 sind jeweils der Träger 4, das Grundteil 83 und der Verdampfer 60 zu sehen.

In Figur 6 wird der Verdampfer 60 in dem Träger 4 angeordnet. Vorteilhaft wird eine Dochtstruktur 19 an der Einlassseite 61 des Verdampfers 60 angeordnet. Vorzugsweise kann der Verdampfer 60 und/oder die Dochtstruktur 19 zur verbesserten Flüssigkeitsdichtigkeit gegen den Träger 4 mit einer Dichtung 120 abgedichtet werden.

Der Träger 4 kann eine elektronische Steuerungsvorrichtung 15 beziehungsweise einen Teil einer elektronischen Steuerungsvorrichtung 15, beispielsweise eine elektronisch identifizierbare Kennung, aufweisen. Die identifizierbare Kennung kann vorzugsweise einen ID-Chip beispielsweise einen SMD EEPROM aufweisen. Die dafür benötigten elektrischen Leitungen 105 sind vorteilhaft wenigstens teilweise in den Träger 4 integriert, wie in Figuren 6 und 7 dargestellt. Die elektrischen Leitungen 105 dienen ebenfalls der elektrischen Versorgung, Steuerung und/oder Regelung des Verdampfers 60.

Der Träger 4 umfasst die elektrische Kontaktierung 140 zur elektrischen Verbindung des Träger 4 und somit des Verdampfereinsatzes 100 mit einem externen Teil, insbesondere ein Basisteil 16 eines Inhalators 10.

Der Träger 4 ist in Figur 6 zur Montage mit dem Grundteil 83 bereitgestellt.

Wie in Figur 7 gezeigt kann der bereitgestellte Träger 4 in das Grundteil 83 eingesetzt werden. Beispielsweise kann der Träger 4 entlang der Längsachse L in das Grundteil 83, insbesondere in den Montageschacht 123 eingesetzt beziehungsweise eingeschoben werden.

In dieser Ausführungsform umfasst die elektrische Kontaktierung 140 drei elektrische Kontakte, die sich im montierten Zustand in Längsrichtung aus den Grundteil 83 erstrecken. Dies kann der elektrischen Kontaktierung des Verdampfereinsatzes 100 mit einem externen Teil zuträglich sein. Drei elektrische Kontakte 110a, 110b, 110c können beispielsweise vorgesehen sein, um eine Erdung, ein Steuersignal und/oder ein Datenübertragungssignal bereitzustellen, siehe auch Figur 9.

In anderen Ausführungsformen können die elektrischen Kontaktierungen 140 beziehungsweise das Grundteil 83 jedoch so ausgebildet sein, dass die elektrischen Kontaktierungen 140 im montierten Zustand im Montageschacht 123 angeordnet sind, um mechanisch geschützt zu sein.

Der Träger 4 hält die Komponenten des Verdampfereinsatzes 100 mechanisch stabil zueinander und verbindet diese wie in Figuren 2 bis 7 zu sehen. Die einzelnen Komponenten umfassen insbesondere den Verdampfer 60, die Dochtstruktur 19 mit vorteilhaft flexiblen Dochtbestandteilen und/oder dem Vlies 119, vorteilhaft elektrische Leitungen 105, elektrische Kontaktierungen 140, vorteilhaft die elektronische Steuerungsvorrichtung 15 und den Luftkanal 130. Dabei können Klemmungen, Klebungen, Schweißungen, Lötungen, Sinterung und/oder andere mechanische Verbindungsvarianten verwendet werden. Die mechanischen Verbindungen zwischen den Komponenten sind so ausgelegt, dass die Verbindungen auch bei der Betriebstemperatur des Verdampfers 60 stabil bleiben und/oder den bei der Liquidverdampfung durch Gasentwicklung entstehenden Drücken standhalten.

Eine Verdampfervorrichtung 1 gemäß Figur 8 umfasst einen blockförmigen, vorzugsweise monolithischer Heizkörper 60 vorzugsweise aus einem elektrisch leitenden Material, insbesondere einem dotierten Halbleitermaterial vorzugsweise Silizium, und einen Träger 4 mit einer Durchgangsöffnung 104 zur flüssigkeitsleitenden Verbindung des Heizkörpers 60 und einem Flüssigkeitsspeicher 18 vorteilhaft über eine in der Durchgangsöffnung 104 angeordnete Dochtstruktur 19. Es ist nicht erforderlich, dass der gesamte Heizkörper 60 aus einem elektrisch leitenden Material besteht. Es kann beispielsweise ausreichen, dass die Oberfläche des Heizkörpers 60 elektrisch leitend, beispielsweise metallisch, beschichtet oder vorzugsweise geeignet dotiert ist. In diesem Fall muss nicht die gesamte Oberfläche beschichtet sein, beispielsweise können metallische oder vorzugsweise nichtmetallische oder nichtmetallisch kaschierte metallische Leiterbahnen auf einem nichtleitenden beziehungsweise halbleitenden Grundkörper vorgesehen sein. Es ist auch nicht zwingend erforderlich, dass der gesamte Heizkörper 60 heizt; es kann beispielsweise ausreichen, wenn ein Abschnitt oder eine Heizschicht des Heizkörpers 60 im Bereich der Austrittsseite 64 heizt.

Der Heizkörper 60 ist mit einer Mehrzahl von Mikrokanälen beziehungsweise Flüssigkeitskanälen 62 versehen, die eine Einlassseite 61 des Heizkörper 60 mit einer Auslassseite 64 des Heizkörper 60 flüssigkeitsleitend verbinden. Die Einlassseite 61 ist über eine nicht in Figur 8 gezeigte Dochtstruktur 19 flüssigkeitsleitend mit dem Flüssigkeitsspeicher 18 verbunden. Die Dochtstruktur 19 dient zur passiven Förderung von Flüssigkeit aus dem Flüssigkeitsspeicher 18 zum Heizkörper 60 mittels Kapillarkräften.

Der mittlere Durchmesser der Flüssigkeitskanäle 62 liegt vorzugsweise im Bereich zwischen 5 µm und 200 µm, weiter vorzugsweise im Bereich zwischen 30 µm und 150 µm, noch weiter vorzugsweise im Bereich zwischen 50 µm und 100 µm. Aufgrund dieser Abmessungen wird vorteilhaft eine Kapillarwirkung erzeugt, so dass an der Einlassseite 61 in einen Flüssigkeitskanal 62 eindringende Flüssigkeit durch den Flüssigkeitskanal 62 nach oben steigt, bis der Flüssigkeitskanal 62 mit Flüssigkeit gefüllt ist. Das Volumenverhältnis von Flüssigkeitskanälen 62 zu Heizkörper 60, das als Porosität des Heizkörper 60 bezeichnet werden kann, liegt beispielsweise im Bereich zwischen 10% und 50%, vorteilhaft im Bereich zwischen 15% und 40%, noch weiter vorteilhaft im Bereich zwischen 20% und 30%, und beträgt beispielsweise 25%.

Die Kantenlängen der mit Flüssigkeitskanälen 62 versehenen Flächen des Heizkörper 60 liegen beispielsweise im Bereich zwischen 0,5 mm und 3 mm, vorzugsweise zwischen 0.5 mm und 1 mm. Die Abmessungen der mit Flüssigkeitskanälen 62 versehenen Flächen des Heizkörpers 60 können beispielsweise betragen: 0,95 mm x 1,75 mm oder 1,9 mm x 1,75 mm oder 1,9 mm x 0,75 mm. Die Kantenlängen des Heizkörpers 60 können beispielsweise im Bereich zwischen 0,5 mm und 5 mm, vorzugsweise im Bereich zwischen 0,75 mm und 4 mm, weiter vorzugsweise im Bereich zwischen 1 mm und 3 mm liegen. Die Fläche des Heizkörpers 60 (chip size) kann beispielsweise 1 mm x 3 mm, 2mm x 2 mm oder 2 mm x 3 mm betragen.

Die Breite b des Heizkörper 60 (siehe Figur 8) liegt vorzugsweise im Bereich zwischen 1 mm und 5 mm, weiter vorzugsweise im Bereich zwischen 2 mm und 4 mm, und beträgt beispielsweise 3 mm. Die Höhe h des Heizkörper 60 (siehe Figur 8) liegt vorzugsweise im Bereich zwischen 0,05 mm und 1 mm, weiter vorzugsweise im Bereich zwischen 0,1 mm und 0,75 mm, noch weiter vorzugsweise im Bereich zwischen 0,2 mm und 0,5 mm und beträgt beispielsweise 0,3 mm. Auch noch kleinere Heizkörper 60 können gefertigt, vorgesehen und funktionsgerecht betrieben werden.

Die Anzahl der Flüssigkeitskanäle 62 liegt vorzugsweise im Bereich zwischen vier und 1000. Auf diese Weise lässt sich der Wärmeeintrag in die Flüssigkeitskanäle 62 optimieren und eine gesicherte hohe Verdampfungsleistung sowie eine ausreichend große Dampfaustrittsfläche realisieren.

Die Flüssigkeitskanäle 62 sind in Form eines quadratischen, rechteckigen, vieleckigen, runden, ovalen oder anders geformten Arrays angeordnet. Das Array kann in Form einer Matrix mit s Spalten und z Zeilen ausgebildet sein, wobei s vorteilhaft im Bereich zwischen 2 und 50 und weiter vorteilhaft im Bereich zwischen 3 und 30 und/oder z vorteilhaft im Bereich zwischen 2 und 50 und weiter vorteilhaft im Bereich zwischen 3 und 30 liegt. Auf diese Weise lässt sich eine effektive und auf einfache Weise herstellbare Anordnung der Flüssigkeitskanäle 62 mit gesichert hoher Verdampfungsleistung realisieren.

Der Querschnitt der Flüssigkeitskanäle 62 kann quadratisch, rechteckig, vieleckig, rund, oval oder anders geformt sein, und/oder sich in Längsrichtung abschnittweise ändern, insbesondere vergrößern, verkleinern oder konstant bleiben.

Die Länge eines oder jedes Flüssigkeitskanals 62 liegt vorzugsweise im Bereich zwischen 100 µm und 1000 µm, weiter vorzugsweise im Bereich zwischen 150 µm und 750 µm, noch weiter vorzugsweise im Bereich zwischen 180 µm und 500 µm und beträgt beispielsweise 300 µm. Auf diese Weise lässt sich eine optimale Flüssigkeitsaufnahme und Portionsbildung bei ausreichend gutem Wärmeeintrag von Heizkörper 60 in die Flüssigkeitskanäle 62 realisieren.

Der Abstand zweier Flüssigkeitskanäle 62 beträgt vorzugsweise mindestens das 1,3-fache des lichten Durchmessers eines Flüssigkeitskanals 62, wobei der Abstand auf die Mittelachsen der beiden Flüssigkeitskanäle 62 bezogen ist. Der Abstand kann bevorzugt das 1,5- bis 5-fache, weiter bevorzugt das 2- bis 4-fache des lichten Durchmessers eines Flüssigkeitskanals 62 betragen. Auf diese Weise lässt sich ein optimaler Wärmeeintrag in den Heizkörper 60 und eine ausreichend stabile Anordnung und Wandstärke der Flüssigkeitskanäle 62 realisieren.

Aufgrund der vorbeschriebenen Merkmale kann der Heizkörper 60 auch als Volumenheizer bezeichnet werden, insbesondere wenn der Heizkörper 60 im Wesentlichen in seinem ganzen Volumen elektrisch leitfähig ausgebildet ist und als Widerstandsheizer ausgebildet ist.

Die Verdampfervorrichtung 1 weist eine vorzugsweise von der Steuerungsvorrichtung 15 steuerbare Heizspannungsquelle 71 auf, die über Elektroden 72 an gegenüberliegenden Seiten des Heizkörpers 60 mit diesem verbunden ist, so dass eine von der Heizspannungsquelle 71 erzeugte elektrische Spannung Uh zu einem Stromfluss durch den Heizkörper 60 führt. Aufgrund des Ohm'schen Widerstands des elektrisch leitenden Heizkörpers 60 führt der Stromfluss zu einer Erhitzung des Heizkörpers 60 und daher zu einer Verdampfung von in den Flüssigkeitskanälen 62 enthaltener Flüssigkeit. Auf diese Weise erzeugter Dampf/Aerosol 6 entweicht zur Auslassseite 64 aus den Flüssigkeitskanälen 62 und wird dem Luftstrom 34 beigemischt, siehe Figur 1. Genauer steuert bei Feststellung eines durch Ziehen des Konsumenten verursachten Luftstroms 34 durch den Luftstromkanal 30 die Steuerungsvorrichtung 15 die Heizspannungsquelle 71 an, wobei durch spontane Erhitzung die in den Flüssigkeitskanälen 62 befindliche Flüssigkeit in Form von Dampf/Aerosol aus den Flüssigkeitskanälen 62 getrieben wird.

Dabei kann die Dauer der einzelnen Verdampfungsschritte bei unterschiedlichen Temperaturen und/oder einem Verdampfen der einzelnen Komponenten der einzelnen Portionen der Flüssigkeit derart kurz gehalten werden und/oder mit einer Ansteuerfrequenz getaktet erfolgen, dass die schrittweise Verdampfung von einem Konsumenten nicht wahrgenommen und trotzdem eine weitgehend homogene, geschmackskonforme, wiederholbar präzise Aerosolbildung gewährleistet werden kann.

Eine elektronische beziehungsweise elektrische Anbindung des Heizkörpers 60 kann beispielsweise über Klemm-, Feder- oder Presskontakte, wirebonding und/oder Löten erfolgen.

Vorzugsweise ist in dem Datenspeicher des Inhalators 10 eine dem verwendeten Flüssigkeitsgemisch angepasste Spannungskurve Uh(t) hinterlegt. Dies ermöglicht es, den Spannungsverlauf Uh(t) dem verwendeten Liquid angepasst vorzugeben, so dass sich die Heiztemperatur des Heizkörpers 60, und damit auch die Temperatur der kapillaren Flüssigkeitskanäle 62, gemäß der bekannten Verdampfungskinetik des jeweiligen Liquids zeitlich über den Verdampfungsvorgang steuern lässt, wodurch optimale Verdampfungsergebnisse erzielbar sind. Die Verdampfungstemperatur liegt vorzugsweise im Bereich zwischen 100 °C und 400 °C, weiter bevorzugt zwischen 150 °C und 350 °C, noch weiter bevorzugt zwischen 190 °C und 290 °C.

Der Heizkörper 60 kann vorteilhaft aus Teilstücken eines Wafers mit Dünnfilmschichttechnologie hergestellt werden, welcher eine Schichtdicke von vorzugsweise kleiner oder gleich 1000 µm, weiter vorzugsweise 750 µm, noch weiter vorzugsweise kleiner oder gleich 500 µm aufweist. Oberflächen des Heizkörpers 60 können vorteilhaft hydrophil sein. Die Auslassseite 64 des Heizkörpers 60 kann vorteilhaft mikrostrukturiert sein bzw. Mikroausnehmungen (micro grooves) aufweisen.

Die Verdampfervorrichtung 1 ist so eingestellt, dass eine Flüssigkeitsmenge vorzugsweise im Bereich zwischen 1 µl und 20 µl, weiter vorzugsweise zwischen 2 µl und 10 µl, noch weiter vorzugsweise zwischen 3 µl und 5 µl, typischerweise 4 µl pro Zug des Konsumenten, zudosiert wird. Vorzugsweise kann die Verdampfervorrichtung 1 hinsichtlich der Flüssigkeits-/Dampfmenge pro Zug, d.h. je Zugdauer von 1 s bis 3 s, einstellbar sein.

Im Folgenden wird beispielhaft der Ablauf des Verdampfungsvorgangs erläutert.

In einem Ausgangszustand ist die Spannungsquelle 71 beziehungsweise der Energiespeicher 14 für den Heizvorgang ausgeschaltet.

Zum Verdampfen von Flüssigkeit 50 wird die Spannungsquelle 14, 71 für den Heizkörper 60 aktiviert. Die Spannung Uh wird dabei so eingestellt, dass die Verdampfungstemperatur in dem Heizkörper 60 und somit in den Flüssigkeitskanälen 62 an das individuelle Verdampfungsverhalten des eingesetzten Flüssigkeitsgemischs angepasst ist. Dies verhindert die Gefahr von lokaler Überhitzung und dadurch Schadstoffentstehung.

Insbesondere kann auch einer unerwünschten differentiellen Verdampfung eines Liquidgemisches entgegengewirkt oder begegnet werden oder eine solche vermieden werden. Ein Liquidgemisch könnte sonst Komponenten aufgrund unterschiedlicher Siedetemperaturen vorschnell im Laufe einer Abfolge von Verdampfungsvorgängen, insbesondere "puffs", verlieren, bevor das Reservoir 18 des Liquids 50 vollständig entleert ist, was beim Betrieb unerwünschte Effekte wie beispielsweise die mangelnde Konstanz der Dosierung bei einem Benutzer nach sich ziehen könnte, insbesondere bei einem pharmazeutisch wirksamen Liquid.

Sobald eine Flüssigkeitsmenge verdampft ist, die dem Volumen der Flüssigkeitskanäle 62 entspricht oder damit in Zusammenhang steht, wird die Heizspannungsquelle 71 deaktiviert. Da die Liquideigenschaften und -menge vorteilhaft exakt bekannt sind und der Heizkörper 60 einen messbaren temperaturabhängigen Widerstand aufweist, kann dieser Zeitpunkt sehr genau bestimmt bzw. gesteuert werden. Die Energieaufnahme der Verdampfervorrichtung 1 lässt sich daher gegenüber bekannten Vorrichtungen reduzieren, da die benötigte Verdampfungsenergie dosierter und damit exakter eingebracht werden kann.

Nach Abschluss des Heizvorgangs sind die Flüssigkeitskanäle 62 überwiegend oder vollständig entleert. Die Heizspannung 71 wird dann so lange ausgeschaltet gehalten, bis mittels Nachförderung von Flüssigkeit durch die Dochtstruktur 19 die Flüssigkeitskanäle 62 wieder aufgefüllt sind. Sobald dies der Fall ist, kann der nächste Heizzyklus durch einschalten der Heizspannung 71 begonnen werden.

Die von der Heizspannungsquelle 71 erzeugte Ansteuerfrequenz des Heizkörpers 60 liegt im Allgemeinen vorteilhaft im Bereich von 1 Hz bis 50 kHz, bevorzugt im Bereich von 30 Hz bis 30 kHz, noch weiter vorteilhaft im Bereich von 100 Hz bis 25 kHz.

Die Frequenz und der Tastgrad der Heizspannung Uh für den Heizkörper 60 sind vorteilhaft an die Eigenschwingung bzw. Eigenfrequenz der Blasenschwingungen während der Blasensiedung angepasst. Vorteilhaft kann die Periodendauer 1/f der Heizspannung daher im Bereich zwischen 5 ms und 50 ms, weiter vorteilhaft zwischen 10 ms und 40 ms, noch weiter vorteilhaft zwischen 15 ms und 30 ms liegen und beispielsweise 20 ms betragen. Je nach Zusammensetzung der verdampften Flüssigkeit 50 können andere als die genannten Frequenzen optimal an die Eigenschwingung bzw. Eigenfrequenz der Blasenschwingungen angepasst sein.

Des Weiteren hat sich gezeigt, dass der durch die Heizspannung Uh erzeugten maximale Heizstrom vorzugsweise nicht mehr als 7 A, weiter vorzugsweise nicht mehr als 6,5 A, noch weiter vorzugsweise nicht mehr als 6 A betragen und optimalerweise im Bereich zwischen 4 A und 6 A liegen sollten, um konzentrierten Dampf bei Vermeidung von Überhitzung zu gewährleisten.

Die Förderrate der Dochtstruktur 19 ist wiederum optimal an die an die Verdampfungsrate des Heizkörpers 60 angepasst, so dass jederzeit ausreichend Flüssigkeit 50 nachgefördert werden kann und ein Leerlaufen des Bereichs vor dem Heizkörper 60 vermieden wird.

Die Verdampfervorrichtung 1 ist vorzugsweise auf der Grundlage von MEMS-Technologie, insbesondere aus Silizium, gefertigt und daher vorteilhaft ein Mikro-Elektro-Mechanisches System.

Vorgeschlagen wird nach dem zuvor Gesagten vorteilhaft ein Schichtaufbau bestehend aus einem vorteilhaft mindestens auf der Einlassseite 61 planaren Heizkörper 60 auf Si-Basis und einer oder mehrerer darunter liegender Kapillarstrukturen 19 mit vorteilhaft unterschiedlicher Porengröße. Die direkt an der Einlassseite 61 des Heizkörpers 60 angeordnete Dochtstruktur 19 verhindert die Bildung von Blasen an der Einlassseite 61 des Heizkörpers 60, da Gasblasen eine weitere Förderwirkung unterbinden und gleichzeitig zu einer (lokalen) Überhitzung des Heizkörpers 60 aufgrund fehlender Kühlung durch nachströmendes Liquid führen.

Figur 9 zeigt einen Längsschnitt durch eine Verdampfer-Tank-Einheit 17. Der Verdampfereinsatz 100 ist eine schematische Darstellung des Verdampfereinsatzes 100 wie in Figuren 2 und 5 beschrieben, wobei der Träger 4 des Verdampfereinsatzes 100 einschalig ist.

Der Verdampfereinsatz 100 weist gegenüberliegend angeordnete Stirnseiten 11, 12 und das um den vom Luftstrom 34 durchströmbaren Luftstromkanal 30 angeordnete Grundteil 83 zwischen den Stirnseiten 11, 12 auf. Entlang des Luftstromkanals 30 und/oder zwischen den Stirnseiten 11, 12 ist die Längsachse L definiert. Das Grundteil 83 erstreckt sich entlang der Längsachse L bzw. des Luftstromkanals 30 zwischen den Stirnseiten 11, 12.

Der Verdampfereinsatz 100 ist patronen- oder hülsenförmig. In diesem Beispiel weist das Grundteil 83 einen abschnittsweise runden Querschnitt auf. Die Längserstreckung entlang des Luftstromkanals 30 ist größer als der Durchmesser des Grundteils 83. Damit ist der Verdampfereinsatz 100 länglich. Vorzugsweise hat der Verdampfereinsatz 100 eine Länge von 0,1 cm bis 10 cm, weiter vorzugsweise 0,5 cm bis 5 cm, beispielsweise von 2 cm und einen Durchmesser von vorzugsweise 0,1 cm bis 1 cm, weiter vorzugsweise 0,25 cm bis 0,75 cm, beispielsweise 0,5 cm. Entlang der Längsachse L des Verdampfereinsatzes 100 weist das Grundteil 83 einen hohlzylinderförmigen Abschnitt und einen sich nicht notwendigerweise verjüngenden Abschnitt mit einem stromabwärts abnehmenden Durchmesser beziehungsweise Querschnitt auf.

Der Luftstrom 34 tritt an der dem Mundende 32 (siehe Figur 1) abgewandten Stirnseite 12 in den Luftstromkanal 30, durchströmt den Luftstromkanal 30 und tritt an der dem Mundende 32 zugewandten Stirnseite 11 aus dem Luftstromkanal 30 aus. In dem Luftkanal 130 wird dem Luftstrom 34 verdampfte Flüssigkeit 50 zugegeben, um dem Konsumenten ein Aerosol bzw. Aerosol-Dampf-Gemisch zu verabreichen.

Der Luftstromkanal 30 umfasst einen als Kamin 40 bezeichneten Abschnitt, der dem Verdampfer 60 zugeordnet ist, und einen vom Verdampfer 60 stromabwärts angeordneten Schlot 41, siehe Figur 9. Der Schlot 41 kann vom Anwender und/oder Hersteller je nach erforderlicher Länge entsprechend gestutzt werden bzw. kürzbar sein.

In diesem Ausführungsbeispiel weist die elektrische Kontaktierung 140 drei elektrische Kontakte 110a, 110b, 110c an einer stromaufwärts des Verdampfers 60 angeordneten Bodenplatte 7 des Verdampfereinsatzes 100 auf.

Die vorzugsweise standardisierte, elektrische Kontaktierung 140 zwischen dem Verdampfereinsatz 1 und beispielsweise einem Basisteil 16 des Inhalators 10 dient beispielsweise der Ansteuerung des oder der Verdampfer 60 und/oder einer Identifizierung der Kartusche über einen vorgesehenen ID-Chip oder eine andere Identifizierungsmethode. Vorzugsweise sind die elektrischen Kontakte 110a, 110b, 110c in einer verdrehsicheren Standard-Anordnung, zum Beispiel in Form von konzentrischen Kontaktpads für Federstifte definiert. Es ist eine kontaktlose Identifizierungsmethode, beispielsweise über RFID und/oder Nahfeldkommunikation (NFC) denkbar.

Die Bodenplatte 7 kann demontierbar oder fest mit dem Grundteil 83 verbunden sein. Die Bodenplatte 7, der Kamin 40 und der Schlot 41 definieren vorzugsweise eine standardisierte Baueinheit, die für verschieden geformte Flüssigkeitsspeichers 18 geeignet ist. Es sind auch Ausführungsformen ohne Bodenplatte 7 denkbar. Die Bodenplatte 7 weist zur Durchströmbarkeit von Luft eine vorteilhaft mittige Öffnung auf. Anstelle von oder zusätzlich zu der in Figur 9 gezeigten Bodenplatte 7 kann der in Figuren 2 und 5 erkennbare Kragen 125 am Grundteil 83 vorgesehen sein.

Figur 9 zeigt die Verdampfer-Tank-Einheit 17 umfassend einen Flüssigkeitsspeicher 18 und den darin eingesetzten Verdampfereinsatz 100. Der Flüssigkeitsspeicher 18 weist ein Gehäuse mit einer Einsetzöffnung 42 und einer Ausgangsöffnung 74 auf, in die ein Verdampfereinsatz 100 anordenbar, insbesondere einschiebbar beziehungsweise einsetzbar ist. Vorteilhaft ist der Verdampfereinsatz 100 entlang der Längsachse L in die Einsetzöffnung 42 einsetzbar beziehungsweise einschiebbar. Optional kann der Flüssigkeitsspeicher 18 einen Montagekanal aufweisen. Der optionale Montagekanal kann in Wirkverbindung mit einer Einsetzöffnung 42 und einer Ausgangsöffnung 74 stehen.

In dem Luftstromkanal 30, beispielsweise im Schlot 41 kann mindestens eine Be- und/oder Entlüftungseinrichtung 117 vorgesehen sein. Die Be- und/oder Entlüftungseinrichtung 117 kann beispielsweise entlang des Schlots 41 eine Mehrzahl von Perforationen aufweisen. Um ein Austritt von Liquid durch die Löcher beziehungsweise Perforationen zu vermeiden, kann der Schlot 41 bzw. das Grundteil 83 durch eine geeignete Materialwahl, die Ausnutzung des Lotuseffekts oder eine Strukturierung der Oberfläche, beispielsweise einer Nanostrukturierung, so beschaffen sein, dass ein möglichst großer Kontaktwinkel zwischen der Oberfläche und dem Liquid besteht und die Oberfläche flüssigkeitsabweisend wird.

Das Grundteil 83 kann auch wenigstens teilweise aus einem semipermeablen Material bestehen, was keine Flüssigkeit 50 in den Luftstromkanal 30 eintreten lässt und gleichzeitig eine Be- und/oder Entlüftung des Flüssigkeitsspeichers 18 erlaubt.

Der Verdampfereinsatz 100 weist vorzugsweise ein Halte- und/oder Befestigungselement 38 zur Befestigung und Halterung des Verdampfereinsatzes 100 in der Einsetzöffnung 42, der Ausgangsöffnung 74 und/oder dem Montagekanal beziehungsweise in dem Flüssigkeitsspeicher 18 auf. Das Halte- und/oder Befestigungselement 38 kann beispielsweise eine Aussparung, Ausbeulung, Nase, Nut und/ oder ein Rastelement aufweisen. Das Halte- und/oder Befestigungselement 38 kann einseitig und/oder nicht-rotationssymmetrisch sein, um eine verdrehsichere Montage des Verdampfereinsatzes 100 im Flüssigkeitsspeicher 18 zu ermöglichen. Das Halte- und/oder Befestigungselement 38 bewirkt die ortsfeste Halterung des Verdampfereinsatzes 100 im Flüssigkeitsspeicher 18 und verhindert ein Verschieben und/oder Verdrehen des Verdampfereinsatzes 100. Das Halte- und/oder Befestigungselement 38 kann, wie in dem Ausführungsbeispiel gezeigt durch die Formung der Mantelseite 31 des Grundteils 83 gebildet sein. Das gezeigte Halte- und/oder Befestigungselement 38 umfasst eine Verjüngung des Grundteils 83 und verhindert ein Verschieben in Richtung der Längsachse L des Verdampfereinsatzes 1.

Das Halte- und/oder Befestigungselement 38 kann den Verdampfereinsatz 100 unlösbar in dem Flüssigkeitsspeicher 18 halten, bspw. durch Verkleben oder Verschweißen, so dass die Verdampfer-Tank-Einheit 17 ein Produkt ist, in dem der Verdampfereinsatz 100 fest und für den Konsumenten unlösbar verbaut ist. Alternativ ist der Einsatz in Offen-Tank-Systemen, die einen durch den Nutzer wiederbefüllbaren Flüssigkeitsspeicher 18 aufweisen, denkbar.

Das Halte- und/oder Befestigungselement 38 kann so gestaltet sein, dass der Verdampfereinsatz 100 in die Einsetzöffnung 42, die Ausgangsöffnung 74 und/oder den Montagekanal bzw. in den Flüssigkeitsspeicher 18 einsetzbar und herausnehmbar ist. Dies erlaubt eine reversible Montage der Verdampfer-Tank-Einheit 17 und ein Auswechseln des Verdampfereinsatzes 100 unter Beibehaltung des Flüssigkeitsspeichers 18, womit der Flüssigkeitsspeicher 18 und/oder der Verdampfereinsatz 100 als Mehrwegteil ausgeführt sein kann. Mit den erläuterten Ausführungsformen ist die Integration des Verdampfereinsatzes 100 in verschiedene Flüssigkeitsspeichers 18 mit unterschiedlichen Tanksystemen denkbar.

Die Grundform des patronenförmigen Verdampfereinsatzes 100 ist dazu eingerichtet, dass der Verdampfereinsatz 100 in verschiedene Flüssigkeitsspeichers 18 mit nur minimalen Anpassungen und vorzugsweise wenigen mechanischen Schnittstellen eingebaut werden kann. Vorteilhaft ist der Verdampfereinsatz 100 mit dem Flüssigkeitsspeicher 18 flüssigkeitsdicht verbunden, um eine Flüssigkeitsleckage an der Verbindungsstelle zu verhindern. Die Verbindung zwischen dem Verdampfereinsatz 100 und dem Flüssigkeitsspeicher 18 kann beispielsweise durch Presspassung, Schweißen und/oder Kleben realisiert werden und/oder eine Dichtung 120 umfassen.

Durch die Integration der Verdampferfunktion und der elektronischen Steuerung in den Verdampfereinsatz 100 sind die funktionellen Anforderungen an den Flüssigkeitsspeicher 18 gering und beschränken sich auf die Aufnahme des Liquids bzw. der Flüssigkeit 50. Dies lässt die Formgebung des Flüssigkeitsspeichers 18 bzw. des Gehäuses offen, da vorzugsweise die einzige Beschränkung der Gehäuseform die Aussparung für die Einsetzöffnung 42, die Ausgangsöffnung 74 und/oder des Montagekanals ist.

Der Flüssigkeitsspeicher 18 kann vorteilhaft von einem Flüssigkeitspufferelement beziehungsweise Liquidzuführelement 33 wenigstens teilweise ausgefüllt oder voll sein. Der Flüssigkeitsöffnung 115 ist das in dem Flüssigkeitsspeicher 18 angeordnetes Liquidzuführelement 33 zugeordnet. Das Liquidzuführelement 33 ist vorteilhaft im Kontakt zu der Dochtstruktur 19 angeordnet. Das Liquidzuführelement 33 ist dazu eingerichtet, Flüssigkeit 50 aus dem Flüssigkeitsspeicher 18 zu speichern und zu der Dochtstruktur 19 zu transportieren. Dies erlaubt eine zuverlässige füllstands-, lage- und/oder orientierungsunabhängige Versorgung der Dochtstruktur 19 mit Flüssigkeit 50 aus dem Flüssigkeitsspeicher 18. Das Liquidzuführelement 33 kann wie die Dochtstruktur 19 aus einem der beschriebenen porösen und/oder kapillaren flüssigkeitsleitenden Materialien bestehen.

An der Mantelseite 31 des Grundteils 83 ist ein Dorn 32 vorgesehen, der zur Halterung des Liquidzuführelements 33 innerhalb eines Flüssigkeitsspeichers 18 vorgesehen sein kann.

Der Dorn 32 kann als Halte- und/oder Befestigungselement 38 dienen und eine verschiebefeste und/oder verdrehsichere Montage des Verdampfereinsatzes 100 im Flüssigkeitsspeicher 18 unterstützen. Der Dorn 32 kann auch vollumfänglich als Lippe an der Mantelseite 31 des Grundteils 83 gebildet sein. Der Dorn 32 beziehungsweise die Lippe ist vorteilhaft schmaler als der Abschnitt des Grundteils mit dem größten Umfang beziehungsweise als der Kragen 125, um eine Montage des Verdampfereinsatzes 100 durch Einschub in den Flüssigkeitsspeicher 18 zu ermöglichen.

In den gezeigten Ausführungsformen ist das Grundteil 83 als Tankinnenteil zum Einsetzen in beispielsweise eine Einsetzöffnung 42 eines Flüssigkeitsspeichers 18 ausgebildet. Der Luftkanal 130 ist wenigstens teilweise von dem Träger 4 ausgebildet. Der Luftkanal 130 kann von der Wandung 81 und dem Träger 4 gebildet sein, siehe Figuren 2 und 3 und/oder von dem Träger 4, beispielsweise durch zwei Schalen 108a, 108b, siehe Figuren 4 und 5. Es ist jedoch auch denkbar, dass der Luftkanal 30 durch das Innere eines Trägers 4, beispielsweise eines einschaligen Trägers 4, verläuft. Dafür kann der Träger 4 entsprechend der Ausbildung des Luftkanals 30 durch Spritzgießen aus Kunststoff hergestellt werden und/oder der Luftkanal 130 kann nach der Herstellung des Trägers 4 in den Träger 4 beispielsweise durch Bohren oder Ätzen eingebracht werden.

## Patentansprüche

1. Verdampfereinsatz (100) für einen Inhalator, insbesondere für ein elektronisches Zigarettenprodukt (10), umfassend
- mindestens einen elektrischen Verdampfer (60) zum Verdampfen von dem Verdampfer (60) zugeführter Flüssigkeit (50) und Zuführen der verdampften Flüssigkeit zu einem Luftstrom (34),
- ein Grundteil (83) mit einer Mantelseite (31), die einen von dem Luftstrom (34) durchströmbaren Luftstromkanal (30) umschließt,
- wobei an der Mantelseite (31) des Grundteils (83) mindestens eine Flüssigkeitsöffnung (115) zum Zuführen von verdampfbarer Flüssigkeit (50) von außen in den Verdampfereinsatz (100) zu dem Verdampfer (60) angeordnet ist, wobei
- der Verdampfereinsatz (100) einen Träger (4) zur Halterung des Verdampfers (60) umfasst, wobei
- der Träger (4) eine Durchgangsöffnung (104) aufweist, die zur Flüssigkeitsversorgung des Verdampfers (60) mit der Flüssigkeitsöffnung (115) korrespondiert,
wobei
- eine sich durch die Durchgangsöffnung (104) erstreckende Dochtstruktur (19) vorgesehen ist,
**dadurch gekennzeichnet, dass** der Träger (4)
- einen Strömungs- und/oder Füllstandssensor; und/oder
- eine elektronische Steuereinrichtung (15) umfasst.

2. Verdampfereinsatz (100) nach Anspruch 1, **dadurch gekennzeichnet, dass**
- der Träger (4) einen Luftkanal (130) zum Führen des Luftstroms (34) ausbildet.

3. Verdampfereinsatz (100) nach Anspruch 2, **dadurch gekennzeichnet, dass**
- der Luftkanal (130) zwischen dem Träger (4) und der Wandung (81) des Verdampfereinsatzes (100) und/oder innerhalb des Trägers (4) ausgebildet ist.

4. Verdampfereinsatz (100) nach einem der vorangehenden Ansprüche, **dadurch gekennzeichnet, dass**
- der Träger (4) eine elektrische Kontaktierung (140) zum elektrischen Kontaktieren des Verdampfereinsatzes (100) und/oder eine elektrische Leitung (105) für den Verdampfer (60) umfasst oder trägt.

5. Verdampfereinsatz (100) nach einem der vorangehenden Ansprüche, **dadurch gekennzeichnet, dass**
- elektrischen Kontakte (110a, 110b, 110c) an einer Stirnseite (11, 12) des Verdampfereinsatzes (100) vorgesehen sind.

6. Verdampfereinsatz (100) nach einem der vorangehenden Ansprüche, **dadurch gekennzeichnet, dass**
- der Träger (4) an einer Wandung (81) des Grundteils (83) abgestützt ist.

7. Verdampfereinsatz (100) nach Anspruch 6, **dadurch gekennzeichnet, dass**
- die Mantelseite (31) wenigstens teilweise die Wandung (81) bildet.

8. Verdampfereinsatz (100) nach einem der vorangehenden Ansprüche, **dadurch gekennzeichnet, dass**
- der Träger (4) einen vom Luftkanal (130) verschiedenen Nebenluftkanal (101) zum Führen eines Nebenluftstroms (102) ausbildet.

9. Verdampfereinsatz (100) nach einem der vorangehenden Ansprüche, **dadurch gekennzeichnet, dass**
- der Träger (4) zur Halterung des Verdampfers (60) ein keramisches Substrat (103) umfasst.

10. Verdampfereinsatz (100) nach einem der vorangehenden Ansprüche, **dadurch gekennzeichnet, dass**
- der Träger (4) aus wenigstens ein oder zwei Schalen (108, 108a, 108b), vorzugsweise aus Kunststoff, gebildet ist.

11. Verdampfereinsatz (100) nach einem der vorangehenden Ansprüche, **dadurch gekennzeichnet, dass**
- der Verdampfereinsatz (100) eine Längsachse (L) aufweist, und eine Auslassseite (64) des Verdampfers (60) parallel zur Längsachse (L) ausgerichtet ist.

12. Verdampfereinsatz (100) nach einem der vorangehenden Ansprüche, **dadurch gekennzeichnet, dass**
- der Träger (4) ein Wärmeleitungselement und/oder ein thermisches Isolierelement umfasst.

13. Verdampfereinsatz (100) nach einem der vorangehenden Ansprüche, **dadurch gekennzeichnet, dass**
- der Träger (4) einen Temperatursensor umfasst.

14. Verdampfereinsatz (100) nach einem der vorangehenden Ansprüche, **dadurch gekennzeichnet, dass** das Grundteil (83) patronen- oder hülsenförmig ist.

15. Verdampfereinsatz (100) nach einem der vorangehenden Ansprüche, **dadurch gekennzeichnet, dass**
- der Verdampfereinsatz (100) in einem externen Teil, insbesondere in einem Flüssigkeitsspeicher (18), anordenbar, insbesondere in dieses einsetzbar oder einschiebbar ist.

16. Verdampfer-Tank-Einheit (17) für ein elektronisches Zigarettenprodukt (10), umfassend
- einen Verdampfereinsatz (100) nach einem der vorangehenden Ansprüche und
- einen Flüssigkeitsspeicher (18), wobei
- der Verdampfereinsatz (100) in den Flüssigkeitsspeicher (18) eingesetzt ist.

17. Elektronisches Zigarettenprodukt (10), umfassend
- eine Verdampfer-Tank-Einheit (17) nach Anspruch 16 und
- ein Basisteil (16) zur Versorgung der Verdampfer-Tank-Einheit (17) mit elektrischer Energie.

## Claims

1. Vaporiser insert (100) for an inhaler, in particular an electronic cigarette product (10), comprising
- at least one electrical vaporiser (60) for vaporising fluid (50) supplied to the vaporiser (60) and for supplying the vaporised fluid to an airflow (34),
- a main part (83) having a lateral face (31) which surrounds an airflow duct (30) through which the airflow (34) can flow,
- at least one fluid opening (115) for supplying vaporisable fluid (50) from the outside into the vaporiser insert (100) and to the vaporiser (60) being arranged on the lateral face (31) of the main part (83), wherein
- the vaporiser insert (100) comprises a support (4) for retaining the vaporiser (60),
- the support (4) comprising a passage opening (104) that corresponds to the fluid opening (115) in order to supply fluid to the vaporiser (60),
wherein
- a wick structure (19) that extends through the passage opening (104) is provided,
**characterised in that** the support (4) comprises
- a flow and/or filling level sensor; and/or
- an electronic controller (15).

2. Vaporiser insert (100) according to claim 1, **characterised in that**
- the support (4) forms an air duct (130) for guiding the airflow (34).

3. Vaporiser insert (100) according to claim 2, **characterised in that**
- the air duct (130) is formed between the support (4) and the wall (81) of the vaporiser insert (100), and/or inside the support (4).

4. Vaporiser insert (100) according to any one of the preceding claims, **characterised in that**
- the support (4) comprises or bears electrical contacting means (140) for electrically contacting the vaporiser insert (100), and/or an electrical lead (105) for the vaporiser (60).

5. Vaporiser insert (100) according to any one of the preceding claims, **characterised in that**
- electrical contacts (110a, 110b, 110c) are provided on an end face (11, 12) of the vaporiser insert (100).

6. Vaporiser insert (100) according to any one of the preceding claims, **characterised in that**
- the support (4) bears against a wall (81) of the main part (83).

7. Vaporiser insert (100) according to claim 6, **characterised in that**
- the lateral face (31) forms the wall (81) at least in part.

8. Vaporiser insert (100) according to any one of the preceding claims, **characterised in that**
- the support (4) forms an auxiliary air duct (101) that is different from the air duct (130) and is intended for guiding an auxiliary airflow (102).

9. Vaporiser insert (100) according to any one of the preceding claims, **characterised in that**
- the support (4) for retaining the vaporiser (60) comprises a ceramic substrate (103).

10. Vaporiser insert (100) according to any one of the preceding claims, **characterised in that**
- the support (4) is formed of at least one or two shells (108, 108a, 108b), preferably made of plastics material.

11. Vaporiser insert (100) according to any one of the preceding claims, **characterised in that**
- the vaporiser insert (100) has a longitudinal axis (L), and an outlet side (64) of the vaporiser (60) is oriented so as to be in parallel with the longitudinal axis (L).

12. Vaporiser insert (100) according to any one of the preceding claims, **characterised in that**
- the support (4) comprises a heat conduction element and/or a thermal insulation element.

13. Vaporiser insert (100) according to any one of the preceding claims, **characterised in that**
- the support (4) comprises a temperature sensor.

14. Vaporiser insert (100) according to any one of the preceding claims, **characterised in that** the main part (83) is cartridge-shaped or sleeve-shaped.

15. Vaporiser insert (100) according to any one of the preceding claims, **characterised in that**
- the vaporiser insert (100) can be arranged in, in particular inserted into or pushed into, an external part, in particular a fluid reservoir (18).

16. Vaporiser-tank unit (17) for an electronic cigarette product (10), comprising
- a vaporiser insert (100) according to any one of the preceding claims, and
- a fluid reservoir (18), wherein
- the vaporiser insert (100) is inserted into the fluid reservoir (18).

17. Electronic cigarette product (10), comprising
- a vaporiser-tank unit (17) according to claim 16, and
- a base part (16) for supplying the vaporiser-tank unit (17) with electrical energy.

## Revendications

1. Insert de vaporisation (100) pour un inhalateur, en particulier pour un produit sous forme de cigarette électronique (10), comprenant :
- au moins un vaporisateur (60) électrique pour vaporiser du liquide (50) amené au vaporisateur (60) et amener le liquide vaporisé à un flux d'air (34),
- une partie de base (83) avec un côté enveloppe (31) qui entoure un conduit de flux d'air (30) pouvant être traversé par le flux d'air (34),
- dans lequel au moins une ouverture pour liquide (115) est disposée sur le côté enveloppe (31) de la partie de base (83) pour l'amenée de liquide (50) vaporisable de l'extérieur dans l'insert de vaporisation (100) vers le vaporisateur (60), dans lequel
- l'insert de vaporisation (100) comprend un support (4) pour maintenir le vaporisateur (60), dans lequel
- le support (4) présente une ouverture de passage (104) qui correspond à l'ouverture pour liquide (115) pour l'alimentation en liquide du vaporisateur (60),
- une structure de mèche (19) s'étendant à travers l'ouverture de passage (104) est prévue,
**caractérisé en ce que** le support (4) comprend
- un capteur de débit et/ou de niveau de remplissage ; et/ou
- un dispositif de commande électronique (15).

2. Insert de vaporisation (100) selon la revendication 1, **caractérisé en ce**
- **que** le support (4) forme un conduit d'air (130) pour guider le flux d'air (34).

3. Insert de vaporisation (100) selon la revendication 2, **caractérisé en ce**
- **que** le conduit d'air (130) est formé entre le support (4) et la paroi (81) de l'insert de vaporisation (100) et/ou à l'intérieur du support (4).

4. Insert de vaporisation (100) selon l'une des revendications précédentes, **caractérisé en ce**
- **que** le support (4) comprend ou porte une connexion électrique (140) pour la mise en contact électrique de l'insert de vaporisation (100) et/ou une ligne électrique (105) pour le vaporisateur (60).

5. Insert de vaporisation (100) selon l'une des revendications précédentes, **caractérisé en ce**
- **que** des contacts électriques (110a, 110b, 110c) sont prévus sur une face frontale (11, 12) de l'insert de vaporisation (100).

6. Insert de vaporisation (100) selon l'une des revendications précédentes, **caractérisé en ce**
- **que** le support (4) s'appuie contre une paroi (81) de la partie de base (83).

7. Insert de vaporisation (100) selon la revendication 6, **caractérisé en ce**
- **que** le côté enveloppe (31) forme au moins partiellement la paroi (81).

8. Insert de vaporisation (100) selon l'une des revendications précédentes, **caractérisé en ce**
- **que** le support (4) forme un conduit d'air secondaire (101) différent du conduit d'air (130) pour guider un flux d'air secondaire (102).

9. Insert de vaporisation (100) selon l'une des revendications précédentes, **caractérisé en ce**
- **que** le support (4) comprend un substrat céramique (103) pour maintenir le vaporisateur (60).

10. Insert de vaporisation (100) selon l'une des revendications précédentes, **caractérisé en ce**
- **que** le support (4) est formé d'au moins une ou deux coques (108, 108a, 108b), de préférence en matière plastique.

11. Insert de vaporisation (100) selon l'une des revendications précédentes, **caractérisé en ce**
- **que** l'insert de vaporisation (100) présente un axe longitudinal (L) et un côté sortie (64) du vaporisateur (60) est orienté parallèlement à l'axe longitudinal (L).

12. Insert de vaporisation (100) selon l'une des revendications précédentes, **caractérisé en ce**
- **que** le support (4) comprend un élément de conduction thermique et/ou un élément d'isolation thermique.

13. Insert de vaporisation (100) selon l'une des revendications précédentes, caractérisé en ce
- le support (4) comprend un capteur de température.

14. Insert de vaporisation (100) selon l'une des revendications précédentes, **caractérisé en ce**
- **que** la partie de base (83) est réalisée en forme de cartouche ou de douille.

15. Insert de vaporisation (100) selon l'une des revendications précédentes, **caractérisé en ce**
- **que** l'insert de vaporisation (100) peut être disposé dans une partie externe, en particulier dans un réservoir de liquide (18), et peut en particulier être inséré ou introduit dans celui-ci.

16. Unité vaporisateur-réservoir (17) pour un produit sous forme de cigarette électronique (10), comprenant :
- un insert de vaporisation (100) selon l'une des revendications précédentes et
- un réservoir de liquide (18), dans laquelle
- l'insert de vaporisation (100) est inséré dans le réservoir de liquide (18).

17. Produit sous forme de cigarette électronique (10), comprenant :
- une unité vaporisateur-réservoir (17) selon la revendication 16 et
- une partie de base (16) pour alimenter l'unité vaporisateur-réservoir (17) en énergie électrique.
